(19) Europäisches Patentamt
European Patent Office
Office européen des brevets

(11) EP 1 020 447 A1

(12) **EUROPEAN PATENT APPLICATION**
published in accordance with Art. 158(3) EPC

(43) Date of publication:
**19.07.2000 Bulletin 2000/29**

(21) Application number: **98941740.7**

(22) Date of filing: **07.09.1998**

(51) Int. Cl.$^7$: **C07D 231/16**, C07D 401/06, C07D 403/06, A01N 43/56, A01N 43/34, A01N 43/72

(86) International application number:
**PCT/JP98/03997**

(87) International publication number:
**WO 99/12910 (18.03.1999 Gazette 1999/11)**

(84) Designated Contracting States:
**CH DE FR GB IT LI**

(30) Priority: **11.09.1997 JP 24652997**
**20.05.1998 JP 13832598**

(71) Applicant:
**Nissan Chemical Industries, Ltd.**
**Chiyoda-ku, Tokyo 101-0054 (JP)**

(72) Inventors:
- **AKIYAMA, Shigeaki,**
  **Nissan Chemical Industries,Ltd**
  **Funabashi-shi, Chiba-ken 274-8507 (JP)**
- **NIKI, Toshio,**
  **Nissan Chemical Industries, Ltd.**
  **Funabashi-shi, Chiba-ken 274-8507 (JP)**
- **UTSUNOMIYA, Tomohisa,**
  **Nissan Chemical Industries,**
  **Funabashi-shi, Chiba-ken 274-8507 (JP)**
- **WATANABE, Junichi,**
  **Nissan Chemical Industries, Ltd**
  **Funabashi-shi, Chiba-ken 274-8507 (JP)**
- **NISHIOKA, Masanori,**
  **Nissan Chemical Industries,Ltd**
  **1470, Ohaza Shiraoka, Shiraoka-cho, (JP)**
- **SUZUKI, Hiroyuki,**
  **Nissan Chemical Industries, Ltd.**
  **1470, Ohaza Shiraoka, Shiraoka-cho, Mi (JP)**
- **HAYASAKA, Fumio,**
  **Nissan Chemical Industries, Ltd.**
  **1470, Ohaza Shiraoka, Shiraoka-cho, Min (JP)**
- **YAMAGISHI, Kazuhiro,**
  **Nissan Chemical Ind., Ltd.**
  **1470, Ohaza Shiraoka, Shiraoka-cho (JP)**

(74) Representative: **HOFFMANN - EITLE**
**Patent- und Rechtsanwälte**
**Arabellastrasse 4**
**81925 München (DE)**

# (54) PYRAZOLE COMPOUNDS AND PLANT DISEASE CONTROL AGENT

(57) Pyrazole compounds represented by the general formula (1); salts of the compounds; and a plant disease control agent containing at least one of the compounds and salts as the active ingredient,

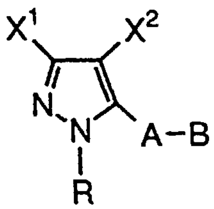

(1)

wherein, R is $C_1$-$C_4$ alkyl,
$X^1$ is halogen,
$X^2$ is H or halogen,
A is a direct bond, $CH_2$, $CH_2CH_2$ and the like,
B is $N_3$, OH, $NH_2$, $C(R^{23})=NR^{24}$, $C(R^{23})=NOR^{24}$ and the like,
$R^{23}$ is H, $C_1$-$C_4$ alkyl and the like,
$R^{24}$ is $C_1$-$C_6$ alkyl, $C_1$-$C_6$ alkenyl and the like.

The compounds are highly effective against plant diseases and are safe for crops.

EP 1 020 447 A1

## Description

Field of the Invention

The present invention relates to novel pyrazole compounds and their salts, as well as plant disease control agents, particularly control agents for rice blast or control agents for diseases of barley or wheat, containing the pyrazole compounds or their salt as active ingredient.

Background Art

Certain pyrazole compounds are known in Japanese Patent Application Laid-Open No. Hei 4-234352 and Japanese Patent Application Laid-Open No. Hei 4-247072, in which use as herbicides or plant growth regulator was disclosed. Further, they are known as fungicide, insecticide and acaricide in Japanese Patent Application Laid-Open No. Hei 5-255268. Still further, they are disclosed as plant disease control agent in Japanese Patent Application Laid-Open No. Hei 8-12510 and Japanese Patent Application Laid-Open No. Hei 9-176125.

However, pyrazole compounds and their salts of the present invention are novel compounds not described in prior papers.

Prior bactericides for agriculture and horticulture are not sufficient for their effects and residue effects due to increase of bacteria having fungicide resistance. Therefore, it has been desired to develop a plant disease control agent having high safety with high efficacy at low application rate, without any phytotoxicity.

Disclosure of the Invention

The Inventors of the present invention have studied variously in order to develop a superior bactericide in consideration of such state. As a result, it has been found that novel pyrazole compounds and their salts have significant activity as a plant disease control agent and that they have significant effect for decreasing phytotoxicity to target crops to accomplish the present invention.

That is, the present invention relates to the following items [1] - [17].

[1] A pyrazole compound of the general formula (1):

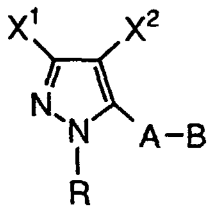

(1)

wherein,

R is $C_1$-$C_4$ alkyl,
$X^1$ is halogen,
$X^2$ is H or halogen,
A is $[CH(R^1)]_g$, $C(R^1)= C(R^2)$ or $C[=C(R^1)(R^2)]$,
g is 0, 1 or 2,
$R^1$ is H, $C_1$-$C_4$ alkyl or phenyl optionally substituted with Ra,
$R^2$ is H, CN, $C_1$-$C_4$ alkyl, $C_1$-$C_4$ alkoxy, $C_1$-$C_4$ alkylthio, $C_1$-$C_4$ alkylsulfinyl, $C_1$-$C_4$ alkylsulfonyl, $C_1$-$C_4$ alkoxycarbonyl or aryl optionally substituted with Ra,
B is, when g is 1 or 2 in the definition of A, $N_3$, OH, $NH_2$, CN, CHO, $OR^3$, $S(O)_d R^3$, $NHS(O)_2R^3$, $N(R^4)OR^3$, $S(O)_2N(R^5)(R^6)$, $N(R^7)(R^8)$, $ON(R^7)(R^8)$, $ON=C(R^9)(R^{10})$, $N(R^4)N(R^5)(R^6)$, $CO_2R^4$, $C(R^{23})=NR^{24}$, $C(R^{23})=NOR^{24}$, $C(R^{23})=NN(R^5)(R^6)$, $C(R^{23})=NN=C(R^9)(R^{10})$,

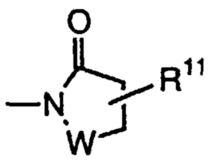

B1 ,

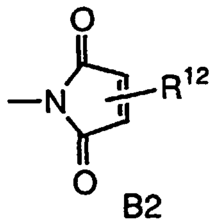

B2 ,

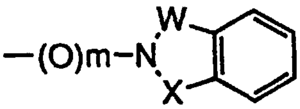

B3 ,

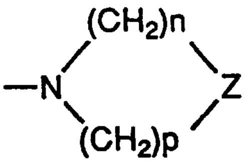

B4 ,

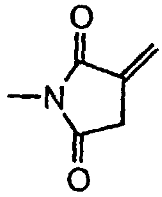 ,

B5

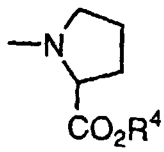 ,

B6

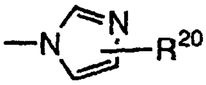 ,

B7

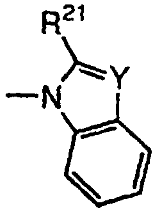 ,

B8

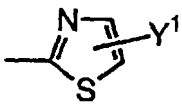 ,

B9

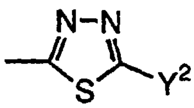 ,

B10

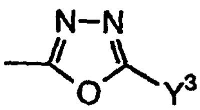 ,

B11

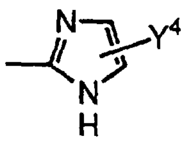 ,

B12

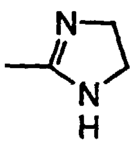 ,

B13

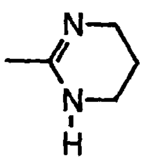 ,

B14

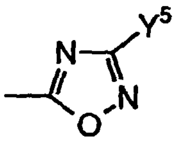 ,

B15

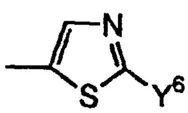 ,

B16

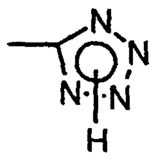 or 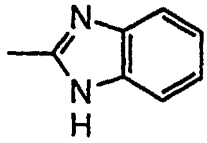

B17 B18

or

B is, when g is 0 in the definition of A, $C(R^{23})=NR^{24}$, $C(R^{23})=NOR^{24}$, $C(R^{23})=NN=(R^5)(R^6)$, $C(R^{23})=NN=C(R^9)(R^{10})$, B9, B10, B11, B12, B13, B14, B15, B16, B17 or B18,

d is 0, 1 or 2,

m is 0 or 1,

n is 1 or 2,

p is 1, 2 or 3,

$R^3$ is $C_1$-$C_{12}$ alkyl, $C_1$-$C_{12}$ haloalkyl, $C_2$-$C_{10}$ alkenyl, $C_2$-$C_{10}$ haloalkenyl, $C_2$-$C_{10}$ alkynyl, $C_3$-$C_{10}$ cycloalkyl or $[C(R^4)(R^{14})]_f$-$R^{15}$,

f is 0, 1 or 2,

$R^4$ is H or $C_1$-$C_4$ alkyl,

$R^5$ and $R^6$ are each independently H, $C_1$-$C_{12}$ alkyl, $C_2$-$C_{10}$ alkenyl, aryl optionally substituted with Ra or benzyl optionally substituted with Ra, or $R^5$ and $R^6$ together are $C_3$-$C_6$ alkylene chain,

$R^7$ is H, $C_1$-$C_{12}$ alkyl, $C_1$-$C_{12}$ haloalkyl, $C_2$-$C_{10}$ alkenyl, $C_3$-$C_{10}$ cycloalkyl or $[C(R^4)(R^{14})]_f$-$R^{15}$,

$R^8$ is $C_1$-$C_{12}$ alkyl, $C_1$-$C_{12}$ haloalkyl, $C_2$-$C_{10}$ alkenyl, $C_3$-$C_{10}$ cycloalkyl, $C_1$-$C_4$ alkylamino-$C_2$-$C_4$ alkyl, di($C_1$-$C_4$ alkyl)amino-$C_2$-$C_4$ alkyl, $C_1$-$C_4$ alkoxy-$C_2$-$C_4$ alkyl or $[C(R^4)(R^{14})]_f$-$R^{15}$, or $R^7$ and $R^8$ together are $C_3$-$C_7$ alkylene chain optionally substituted with OH or $C_1$-$C_4$ alkyl,

$R^9$ and $R^{10}$ are each independently H, $C_1$-$C_{12}$ alkyl, $C_2$-$C_{10}$ alkenyl, $C_3$-$C_{10}$ cycloalkyl, aryl optionally substituted with Ra or benzyl optionally substituted with Ra, or $R^9$ and $R^{10}$ together are $C_3$-$C_6$ alkylene chain,

$R^{11}$ is H or $C_1$-$C_6$ alkyl,

W is direct bond, C(O), $SO_2$, $CH_2$, $CH_2CH_2$ or $CH_2CH_2CH_2$,

$R^{12}$ is H, halogen or $C_1$-$C_6$ alkyl,

X is C(O), $SO_2$, $CH_2$, $CH_2CH_2$, or $CH_2CH_2CH_2$,

Z is O, S, $CH(CH_3)OCH(CH_3)$, CH=CH, $N(R^{13})$ or $CH(CH_3)N(R^{13})CH(CH_3)$,

Y is N or $C(R^{22})$,

$R^{13}$ is H, CHO, $C_1$-$C_6$ alkyl, $C_2$-$C_{10}$ alkenyl, $C_1$-$C_6$ alkoxycarbonyl, $C_3$-$C_{10}$ cycloalkyl, $C_1$-$C_6$ alkylcarbonyl, aryl or benzyl,

$R^{14}$ is H, $C_1$-$C_6$ alkyl, $C_1$-$C_6$ alkylthio-$C_2$-$C_4$ alkyl, aryl optionally substituted with Ra or benzyl optionally substituted with Ra,

$R^{15}$ is aryl optionally substituted with Rb, CN, $C(Q)R^{16}$, $C(Q)OR^{17}$ or $C(Q)N(R^{18})(R^{19})$,

Q is O or S,

$R^{16}$ is H, $C_1$-$C_{12}$ alkyl, $C_1$-$C_{12}$ haloalkyl, $C_2$-$C_{10}$ alkenyl, $C_3$-$C_{10}$ cycloalkyl, aryl optionally substituted with Rb or benzyl optionally substituted with Rb,

$R^{17}$ is H, $C_1$-$C_6$ alkyl, $C_2$-$C_{10}$ alkenyl, aryl optionally substituted with Ra or benzyl optionally substituted with Ra,

$R^{18}$ and $R^{19}$ each independently H, $C_1$-$C_6$ alkyl, aryl optionally substituted with Ra or benzyl optionally substituted with Ra, or $R^{18}$ and $R^{19}$ together are $C_3$-$C_6$ alkylene chain,

$R^{20}$ is H, halogen, $C_1$-$C_4$ alkyl or phenyl,

$R^{21}$ is H, halogen, $C_1$-$C_4$ alkyl or $C_1$-$C_4$ alkylthio,

$R^{22}$ is H, halogen or $C_1$-$C_4$ alkyl,

$R^{23}$ is H, $C_1$-$C_4$ alkyl or phenyl optionally substituted with Ra,

$R^{24}$ is H, $C_1$-$C_6$ alkyl, $C_2$-$C_{10}$ alkenyl, $C_2$-$C_{10}$ haloalkenyl, $CH_2CO_2R^4$, phenyl optionally substituted with Ra or benzyl optionally substituted with Ra,

$Y^1$ is H, $C_1$-$C_4$ alkyl, $C_1$-$C_4$ alkoxycarbonyl or phenyl optionally substituted with Ra,

$Y^2$ is H, $C_1$-$C_4$ alkyl or phenyl optionally substituted with Ra,

$Y^3$ is H, $C_1$-$C_4$ alkyl, SH, $C_1$-$C_4$ alkylthio or phenyl optionally substituted with Ra,

$Y^4$ is H, $C_1$-$C_4$ alkyl or phenyl optionally substituted with Ra,
$Y^5$ is H, $C_1$-$C_4$ alkyl, $C_1$-$C_4$ haloalkyl or phenyl optionally substituted with Ra,
$Y^6$ is H, $NH_2$, $C_1$-$C_4$ alkyl or phenyl optionally substituted with Ra,
Ra, being same or different, is 1 - 5 substituent(s) selected from halogen, $NO_2$, CN, $C_1$-$C_4$ alkyl or $C_1$-$C_4$ alkoxy,
Rb, being same or different, is 1 - 5 substituent(s) selected from halogen, $NO_2$, CN, $NH_2$, OH, $C_1$-$C_4$ alkyl, $C_1$-$C_4$ alkoxy, $C_1$-$C_4$ haloalkyl, carboxyl or $C_1$-$C_4$ alkoxycarbonyl,
aryl is phenyl or naphthyl.

[2] A pyrazole compound according to the above-mentioned [1], wherein $X^1$ is Cl or Br, $X^2$ is H or Cl, R is methyl, ethyl, n- or i-propyl or n-butyl.
[3] A pyrazole compound according to the above-mentioned [1], wherein $X^1$ is Cl or Br, $X^2$ is H or Cl, R is methyl, ethyl, n- or i-propyl or n-butyl, A is $[CH(R^1)]_g$ or $C(R^1)=C(R^2)$.
[4] A pyrazole compound according to the above-mentioned [1], wherein $X^1$ is Cl or Br, $X^2$ is H or Cl, R is methyl, A is $[CH(R^1)]_g$ or $C(R^1)=C(R^2)$.
[5] A pyrazole compound according to the above-mentioned [1], wherein $X^1$ is Cl, $X^2$ is H or Cl, R is methyl, A is $[CH(R^1)]_g$.
[6] A pyrazole compound according to the above-mentioned [1], wherein $X^1$ is Cl, $X^2$ is H or Cl, R is methyl, A is $[CH(R^1)]_g$, g is 1, $R^1$ is H or methyl.
[7] A pyrazole compound according to the above-mentioned [1], wherein $X^1$ is Cl, $X^2$ is H or Cl, R is methyl, A is $CH_2$.
[8] A pyrazole compound according to the above-mentioned [1], wherein $X^1$ is Cl, $X^2$ is H or Cl, R is methyl, A is $CH_2$, B is $NH_2$, OH, $OR^3$, $S(O)_dR^3$, $NHS(O)_2R^3$, $N(R^4)OR^3$, $S(O)_2N(R^5)(R^6)$, $N(R^7)(R^8)$, $ON(R^7)(R^8)$, $ON=C(R^9)(R^{10})$, $N(R^4)N(R^5)(R^8)$, B3, B4 or B7.
[9] A pyrazole compound according to the above-mentioned [1], wherein $X^1$ is Cl, $X^2$ is H, R is methyl, A is $CH_2$, B is $NH_2$, OH, $OR^3$, $N(R^7)(R^8)$, $ON=C(R^9)(R^{10})$, $N(R^4)N(R^5)(R^6)$, B3, B4 or B7.
[10] A pyrazole compound according to the above-mentioned [1], wherein $X^1$ is Cl, $X^2$ is H, R is methyl, A is $CH_2$, B is $NH_2$, $N(R^7)(R^8)$, $N(R^4)N(R^5)(R^6)$, B3, B4 or B7.
[11] A pyrazole compound according to the above-mentioned [1], wherein $X^1$ is Cl, $X^2$ is H, R is methyl, A is $CH_2$, B is $N(R^7)(R^8)$ or B4.
[12) A pyrazole compound according to the above-mentioned [1], wherein $X^1$ is Cl, $X^2$ is H, R is methyl, A is $CH_2$, B is $NH_2$ or $N(R^7)(R^8)$, $R^7$ is H, methyl, ethyl, n- or i-propyl, n-, i-, s- or t-butyl, allyl, phenyl or benzyl, $R^8$ is methyl, ethyl, n- or i-propyl, n-, i-, s- or t-butyl, allyl, phenyl or benzyl, or $R^7$ and $R^8$ together are $(CH_2)_4$, $(CH_2)_5$ or $(CH_2)_6$.
[13] A pyrazole compound according to the above-mentioned [1], wherein $X^1$ is Cl, $X^2$ is H, R is methyl, A is $CH_2$, B is B4, n is 1 or 2, p is 1 or 2, Z is CH=CH, $N(R^{13})$ or $CH(CH_3)N(R^{13})CH(CH_3)$, $R^{13}$ is H, methyl, ethyl, n- or i-propyl, allyl, benzyl or phenyl.
[14] A pyrazole compound according to the above-mentioned [1], wherein $X^1$ is Cl, $X^2$ is H, R is methyl, A is $CH_2$, B is $NH_2$, methylamino, ethylamino, dimethylamino, diethylamino, N-ethyl-n-propylamino, n-propylamino, i-propylamino, di(n-propyl)amino, n-butylamino, i-butylamino, s-butylamino, t-butylamino, di(i-butyl)amino, allylamino, diallylamino, anilino, benzylamino, pyrrolidino, piperidino, hexamethyleneimino, piperazino, N-methylpiperazino, N-ethylpiperazino, N-(n-propyl)piperazino, N-allylpiperazino, N-phenylpiperazino, N-benzylpiperazino or 1 2,3,6-tetrahydropyridino.
[15] A pyrazole compound according to the above-mentioned [1] selected from the group consisting of

(1) 3-chloro-1-methyl-5-(dimethylamino)methyl pyrazole,
(2) 3-chloro-1-methyl-5-(diethylamino)methyl pyrazole,
(3) 3-chloro-1-methyl-5-(N-ethyl-n-propylamino)methyl pyrazole,
(4) 3-chloro-1-methyl-5-(n-propylamino)methyl pyrazole,
(5) 3-chloro-1-methyl-5-(i-butylamino)methyl pyrazole,
(6) 3-chloro-1-methyl-5-(s-butylamino)methyl pyrazole,
(7) 3-chloro-1-methyl-5-(di-i-butylamino)methyl pyrazole,
(8) 3-chloro-1-methyl-5-(allylamino)methyl pyrazole,
(9) 3-chloro-1-methyl-5-(diallylamino)methyl pyrazole,
(10) 3-chloro-1-methyl-5-(benzylamino)methyl pyrazole,
(11) 3-chloro-1-methyl-5-(pyrrolidino)methyl pyrazole,
(12) 3-chloro-1-methyl-5-(hexamethyleneimino)methyl pyrazole,
(13) 3-chloro-1-methyl-5-( 1-piperadino)methyl pyrazole,
(14) 3-chloro-1-methyl-5-(4-methyl-1-piperadino)methyl pyrazole,

(15) 3-chloro-1-methyl-5-(4-ethyl-1-piperadino)methyl pyrazole,
(16) 3-chloro-1-methyl-5-(4-allyl-1-piperadino)methyl pyrazole,
(17) 3-chloro-1-methyl-5-(4-phenyl-1-piperadino)methyl pyrazole,
(18) 3-chloro-1-methyl-5-(4-benzyl-1-piperadino)methyl pyrazole,
(19) (3-chloro-1-methylpyrazol-5-yl)methylamine,
(20) 3-chloro-1-methyl-5-(methylamino)methyl pyrazole and
(21) 3-chloro-1-methyl-5-(ethylamino)methyl pyrazole.

[16] A hydrochloride, hydrobromide, hydroiodide, formate, acetate or oxalate of a pyrazole compound according to one of the above-mentioned [1] - [15].
[17] A Plant disease control agent containing at least one compound or its salt according to one of the above-mentioned [1] - [16] as active ingredient.

Best Mode for carrying out the Invention

In the compounds of the general formula (1) according to the invention;
As for $C_1$-$C_4$ alkyl in R, methyl, ethyl, n- or i-propyl, n-butyl and the like may be mentioned, and methyl is particularly preferable.
As for $X^1$, F, Cl, Br and I may be mentioned, and Cl and Br are preferable. Cl is more preferable.
As for $X^2$, H, Cl, Br and I may be mentioned, and H is particularly preferable.
As for $R^1$, H, methyl, ethyl, n- or i-propyl, n-or t-butyl and phenyl may be mentioned, and H is particularly preferable.
As for $C_1$-$C_4$ alkyl in $R^2$, methyl, ethyl and the like may be mentioned. As for $C_1$-$C_4$ alkoxy, methoxy, ethoxy and the like may be mentioned. As for $C_1$-$C_4$ alkylthio, methylthio may be mentioned. As for $C_1$-$C_4$ alkylsulfinyl, methylsulfinyl may be mentioned. As for $C_1$-$C_4$ alkylsulfonyl, methylsulfonyl may be mentioned. As for $C_1$-$C_4$ alkoxycarbonyl, methoxycarbonyl, ethoxycarbonyl and the like may be mentioned. As for aryl, phenyl may be mentioned.
As for A, direct bond, $CH_2$, $CH_2CH_2$, CH(Me), CH(Ph), CH=CH, C(=$CH_2$) and the like may be mentioned, and $CH_2$ is preferable.
As for $C_1$-$C_4$ alkyl in $R^3$, $R^4$, $R^5$, $R^6$, $R^7$, $R^8$, $R^9$, $R^{10}$, $R^{11}$, $R^{12}$, $R^{13}$, $R^{14}$, $R^{16}$, $R^{17}$, $R^{18}$, $R^{19}$, $R^{23}$ and $R^{24}$, methyl, ethyl, n- or i-propyl, n- or t-butyl and the like may be mentioned. As for $C_1$-$C_6$alkyl, methyl, ethyl, n- or i-propyl, n- or t-butyl, pentyl, hexyl and the like may be mentioned. As for $C_1$-$C_{12}$ alkyl, methyl, ethyl, n- or i-propyl, n-, i-, s- or t-butyl, pentyl, hexyl, heptyl, octyl, nonyl, decyl, dodecyl and the like may be mentioned. As for $C_1$-$C_{12}$ haloalkyl, chloromethyl, dichloromethyl, trichloromethyl, trifluoromethyl, trifluoroethyl, chloropropyl and the like may be mentioned. As for $C_2$-$C_{10}$ alkenyl, vinyl, propenyl and butenyl and the like may be mentioned. As for $C_2$-$C_{10}$ haloalkenyl, chloropropenyl and the like may be mentioned. As for $C_2$-$C_{10}$ alkynyl, ethenyl, propargyl and the like may be mentioned. As for $C_3$-$C_{10}$ cycloalkyl, cyclopropyl, cyclobutyl, cyclopentyl, cyclohexyl, cycloheptyl, cyclooctyl and the like may be mentioned. As for aryl, phenyl, naphthyl and the like may be mentioned. As for $C_1$-$C_4$ alkoxy-$C_2$-$C_4$ alkyl, methoxyethyl, ethoxyethyl, methoxypropyl, ethoxypropyl and the like may be mentioned. As for $C_1$-$C_6$ alkylcarbonyl, acetyl, propionyl, butanoyl and the like may be mentioned. As for aryl, phenyl, naphthyl and the like may be mentioned. As for $C_1$-$C_4$ alkylamino-$C_2$-$C_4$ alkyl, methylaminoethyl, ethylaminoethyl, methylaminopropyl, ethylaminopropyl and the like may be mentioned. As for di($C_1$-$C_4$ alkyl)amino-$C_2$-$C_4$ alkyl, dimethylaminoethyl, diethylaminoethyl, dimethylaminopropyl, diethylaminopropyl and the like may be mentioned.
As for $C_3$-$C_6$ alkylene and $C_3$-$C_7$ alkylene in $R^5$ and $R^6$, $R^7$ and $R^8$, $R^9$ and $R^{10}$, and $R^{18}$ and $R^{19}$, tetramethylene, pentamethylene, hexamethylene and the like may be mentioned. Further, as for $C_1$-$C_4$ alkyl for substitution of the alkylene, methyl may be mentioned.
As for $C_1$-$C_6$ alkylthio-$C_2$-$C_4$ alkyl in $R^{14}$, methylthioethyl and the like may be mentioned.
As for halogen in $R^{20}$, $R^{21}$ and $R^{22}$, F, Cl, Br and I may be mentioned. As for $C_1$-$C_4$ alkyl, methyl, ethyl, n- or i-propyl, n- or t-butyl and the like may be mentioned.
As for $C_1$-$C_4$alkylthio in $R^{21}$, methylthio, ethylthio, n- or i-propylthio and the like may be mentioned.
As for $C_1$-$C_4$ alkyl in $Y^1$, $Y^2$, $Y^3$, $Y^4$, $Y^5$ and $Y^6$, methyl, ethyl, n- or i-propyl, n-or t-butyl and the like may be mentioned.
As for $C_1$-$C_4$ alkoxycarbonyl in $Y^1$, methoxycarbonyl, ethoxycarbonyl and the like may be mentioned.
As for $C_1$-$C_4$ alkylthio in $Y^3$ , methylthio may be mentioned.
As for $C_1$-$C_4$ haloalkyl in $Y^5$, chloromethyl may be mentioned.
In Ra and Rb, as for halogen atoms, F, Cl, Br and I may be mentioned. As for $C_1$-$C_4$alkyl, methyl, ethyl, n- or i-propyl, n- or t-butyl and the like may be mentioned. As for $C_1$-$C_4$ alkoxy, methoxy, ethoxy and the like may be mentioned.
In Rb, as for $C_1$-$C_4$ haloalkyl, trifluoromethyl and the like may be mentioned. As for $C_1$-$C_4$ alkoxycarbonyl,

methoxycarbonyl, ethoxycarbonyl and the like may be mentioned.

The compounds of the formula (1) according to the invention are shown in the following Table 1 to Table 4. However, the compounds according to the invention are not limited thereto.

In the Tables, Me denotes methyl group, Et denotes ethyl group, Pr denotes propyl group, Bu denotes butyl group, Pen denotes pentyl group, Hex denotes hexyl group, Hep denotes heptyl group, Oct denotes octyl group, Non denotes nonyl group, Dec denotes decyl group, Dod denotes dodecyl group, Ph denotes phenyl group, Nap denotes naphthyl group, i denotes iso, s denotes secondary, cyc denotes cyclo, and t denotes tertiary.

Table 1

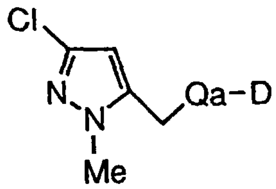 ,

(a)

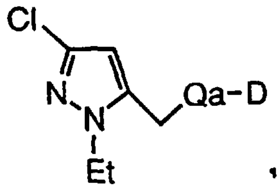 ,

(b)

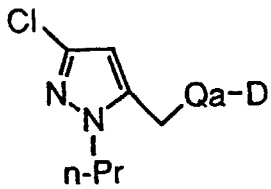 ,

(c)

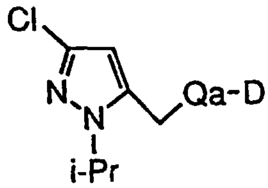 ,

(d)

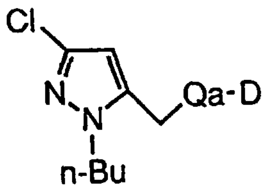 ,

(e)

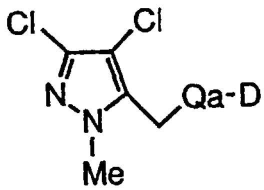 ,

(f)

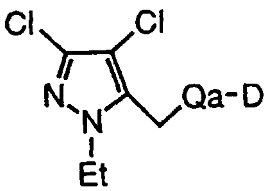 ,

(g)

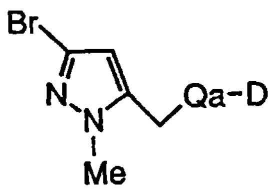 ,

(h)

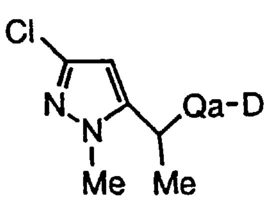

(i)

or

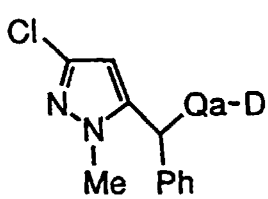

(j)

| Compound No. | Qa | D |
|---|---|---|
| 1-1 | 0 | H |
| 1-2 | 0 | Me |
| 1-3 | 0 | Et |
| 1-4 | 0 | n-Pr |
| 1-5 | 0 | i-Pr |
| 1-6 | 0 | n-Bu |
| 1-7 | 0 | s-Bu |
| 1-8 | 0 | t-Bu |
| 1-9 | 0 | n-Pen |
| 1-10 | 0 | n-Hex |
| 1-11 | 0 | n-Hep |
| 1-12 | 0 | n-Oct |
| 1-13 | 0 | n-Non |
| 1-14 | 0 | n-Dec |
| 1-15 | 0 | n-Dod |
| 1-16 | 0 | cyc-Pr |
| 1-17 | 0 | cyc-Bu |
| 1-18 | 0 | cyc-Pen |
| 1-19 | 0 | cyc-Hex |
| 1-20 | 0 | cyc-Hep |
| 1-21 | 0 | $CH_2=CH_2$ |
| 1-22 | 0 | $CH_2CH=CH_2$ |
| 1-23 | 0 | $CH_2CH=CHCl$ |
| 1-24 | 0 | $CH_2C\equiv CH$ |
| 1-25 | 0 | $C(Me)_2C\equiv CH$ |
| 1-26 | 0 | Ph |
| 1-27 | 0 | 2-Cl-Ph |
| 1-28 | 0 | 3-Cl-Ph |
| 1-29 | 0 | 4-Cl-Ph |
| 1-30 | 0 | 2-Me-Ph |
| 1-31 | 0 | 3-Me-Ph |
| 1-32 | 0 | 4-Me-Ph |
| 1-33 | 0 | 2-CN-Ph |
| 1-34 | 0 | 3-CN-Ph |
| 1-35 | 0 | 4-CN-Ph |
| 1-36 | 0 | 2-$CO_2H$-Ph |
| 1-37 | 0 | 3-$CO_2H$-Ph |
| 1-38 | 0 | 4-$CO_2H$-Ph |
| 1-39 | 0 | 2-$CO_2Me$-Ph |

Table 1 continued

| Compound No. | Q a | D |
|---|---|---|
| 1-40 | O | 3-$CO_2$Me-Ph |
| 1-41 | O | 4-$CO_2$Me-Ph |
| 1-42 | O | 2-$NO_2$-Ph |
| 1-43 | O | 3-$NO_2$-Ph |
| 1-44 | O | 4-$NO_2$-Ph |
| 1-45 | O | 4-$NH_2$-Ph |
| 1-46 | O | 2-$CF_3$-Ph |
| 1-47 | O | 3-$CF_3$-Ph |
| 1-48 | O | 4-$CF_3$-Ph |
| 1-49 | O | 4-HO-Ph |
| 1-50 | O | 1-Nap |
| 1-51 | O | 2-Nap |
| 1-52 | O | $CH_2$Ph |
| 1-53 | O | CH(Me)Ph |
| 1-54 | O | $CHPh_2$ |
| 1-55 | OCO | Me |
| 1-56 | OCO | Et |
| 1-57 | OCO | n-Pr |
| 1-58 | OCO | t-Bu |
| 1-59 | OCO | $CF_3$ |
| 1-60 | OCO | $CH_2Cl$ |
| 1-61 | OCO | $CHCl_2$ |
| 1-62 | OCO | $CCl_3$ |
| 1-63 | OCO | cyc-Pr |
| 1-64 | OCO | cyc-Pen |
| 1-65 | OCO | cyc-Hex |
| 1-66 | OCO | Ph |
| 1-67 | OCO | 2-Cl-Ph |
| 1-68 | OCO | 3-Cl-Ph |
| 1-69 | OCO | 4-Cl-Ph |
| 1-70 | OCO | 2-Me-Ph |
| 1-71 | OCO | 3-Me-Ph |
| 1-72 | OCO | 4-Me-Ph |
| 1-73 | OCO | 2-$CF_3$-Ph |
| 1-74 | OCO | 3-$CF_3$-Ph |
| 1-75 | OCO | 4-$CF_3$-Ph |
| 1-76 | OCO | $CH_2$Ph |
| 1-77 | OCO | $CH_2$(4-Cl-Ph) |

Table 1 continued

| Compound No. | Q a | D |
|---|---|---|
| 1-78 | $OCO_2$ | H |
| 1-79 | $OCO_2$ | Me |
| 1-80 | $OCO_2$ | Et |
| 1-81 | $OCO_2$ | n-Pr |
| 1-82 | $OCO_2$ | n-Bu |
| 1-83 | $OCO_2$ | $CH_2CH=CH_2$ |
| 1-84 | $OCO_2$ | Ph |
| 1-85 | $OCO_2$ | $CH_2Ph$ |
| 1-86 | OCO | $NH_2$ |
| 1-87 | OCO | NHMe |
| 1-88 | OCO | NHEt |
| 1-89 | OCO | NH(n-Pr) |
| 1-90 | OCO | NH(n-Bu) |
| 1-91 | OCO | $NMe_2$ |
| 1-92 | OCO | $NEt_2$ |
| 1-93 | OCO | NHPh |
| 1-94 | OCO | NH(4-Cl-Ph) |
| 1-95 | OCO | NH(4-Me-Ph) |
| 1-96 | OCO | $NH(CH_2Ph)$ |
| 1-97 | OCO | NHCH(Me)Ph |
| 1-98 | OCO | N(Me)Ph |
| 1-99 | OCO | T1 |
| 1-100 | OCO | T2 |
| 1-101 | O | $NH_2$ |
| 1-102 | O | NHMe |
| 1-103 | O | $NMe_2$ |
| 1-104 | O | NHPh |
| 1-105 | O | NHCOMe |
| 1-106 | O | NHCOEt |
| 1-107 | O | NHCOPh |
| 1-108 | O | NHCO(4-Cl-Ph) |
| 1-109 | O | $NHCOCH_2Ph$ |
| 1-110 | O | $NHCO_2Me$ |
| 1-111 | O | $NHCO_2Et$ |
| 1-112 | O | $NH(CH_2Ph)$ |
| 1-113 | O | T3 |
| 1-114 | ON= | $CH_2$ |
| 1-115 | ON= | CHMe |

Table 1 continued

| Compound No. | Q a | D |
|---|---|---|
| 1-116 | ON= | CHEt |
| 1-117 | ON= | CH(n-Pr) |
| 1-118 | ON= | CH(n-Bu) |
| 1-119 | ON= | CH(cyc-Pen) |
| 1-120 | ON= | CH(cyc-Hex) |
| 1-121 | ON= | CHPh |
| 1-122 | ON= | CH(2-Cl-Ph) |
| 1-123 | ON= | CH(3-Cl-Ph) |
| 1-124 | ON= | CH(4-Cl-Ph) |
| 1-125 | ON= | $CH(CH_2Ph)$ |
| 1-126 | ON= | $C(Me)_2$ |
| 1-127 | ON= | C(Me)(Et) |
| 1-128 | ON= | C(Me)(n-Pr) |
| 1-129 | ON= | C(Me)(i-Pr) |
| 1-130 | ON= | C(Me)(n-Bu) |
| 1-131 | ON= | C(Me)(t-Bu) |
| 1-132 | ON= | C(Me)(Ph) |
| 1-133 | ON= | C(Me)(2-Cl-Ph) |
| 1-134 | ON= | C(Me)(3-Cl-Ph) |
| 1-135 | ON= | C(Me)(4-Cl-Ph) |
| 1-136 | ON= | C(Me)(2-Me-Ph) |
| 1-137 | ON= | C(Me)(3-Me-Ph) |
| 1-138 | ON= | C(Me)(4-Me-Ph) |
| 1-139 | ON= | $C(Me)(CH=CH_2)$ |
| 1-140 | ON= | $C(Me)(CH_2CH=CH_2)$ |
| 1-141 | ON= | C(Me)(cyc-Pr) |
| 1-142 | ON= | C(Me)(cyc-Pen) |
| 1-143 | ON= | C(Me)(cyc-Hex) |
| 1-144 | ON= | $C(Me)(CH_2Ph)$ |
| 1-145 | ON= | $C(Me)(CH_2(4\text{-}Cl\text{-}Ph))$ |
| 1-146 | ON= | cyc-Pr |
| 1-147 | ON= | cyc-Bu |
| 1-148 | ON= | cyc-Pen |
| 1-149 | ON= | cyc-Hex |
| 1-150 | $OCH_2$ | $CO_2Me$ |
| 1-151 | $OCH_2$ | $CO_2Et$ |
| 1-152 | $OCH_2$ | $CO_2(n\text{-}Pr)$ |
| 1-153 | $OCH_2$ | $CO_2(n\text{-}Bu)$ |

Table 1continued

| Compound No. | Q a | D |
|---|---|---|
| 1-154 | $OCH_2$ | $CO_2Ph$ |
| 1-155 | $OCH_2$ | $CO_2CH_2Ph$ |
| 1-156 | OCH(Me) | $CO_2Me$ |
| 1-157 | OCH(Me) | $CO_2Et$ |
| 1-158 | OCH(Me) | $CO_2$(n-Pr) |
| 1-159 | OCH(Me) | $CO_2$(n-Bu) |
| 1-160 | OCH(Me) | $CO_2Ph$ |
| 1-161 | OCH(Me) | $CO_2CH_2Ph$ |
| 1-162 | $OCH_2$ | $CONH_2$ |
| 1-163 | $OCH_2$ | CONHMe |
| 1-164 | $OCH_2$ | CONHEt |
| 1-165 | $OCH_2$ | CONH(n-Pr) |
| 1-166 | $OCH_2$ | CONH(n-Bu) |
| 1-167 | $OCH_2$ | CONHPh |
| 1-168 | $OCH_2$ | CONH($CH_2Ph$) |
| 1-169 | $OCH_2$ | $CONMe_2$ |
| 1-170 | $OCH_2$ | CON(Me)(Et) |
| 1-171 | $OCH_2$ | CON(Me)(Ph) |
| 1-172 | $OCH_2$ | T4 |
| 1-173 | $OCH_2$ | T5 |
| 1-174 | $OCH_2$ | T6 |
| 1-175 | S | H |
| 1-176 | S | Me |
| 1-177 | S | Et |
| 1-178 | S | n-Pr |
| 1-179 | S | n-Bu |
| 1-180 | S | t-Bu |
| 1-181 | S | $CH_2CH=CH_2$ |
| 1-182 | S | Ph |
| 1-183 | S | 2-Cl-Ph |
| 1-184 | S | 3-Cl-Ph |
| 1-185 | S | 4-Cl-Ph |
| 1-186 | S | 2-Me-Ph |
| 1-187 | S | 3-Me-Ph |
| 1-188 | S | 4-Me-Ph |
| 1-189 | S | 2-$CO_2$Me-Ph |
| 1-190 | S | 3-$CO_2$Me-Ph |
| 1-191 | S | 4-$CO_2$Me-Ph |

Table 1 continued

| Compound No. | Q a | D |
|---|---|---|
| 1-192 | S | $CH_2Ph$ |
| 1-193 | S | $CH_2$(4-Cl-Ph) |
| 1-194 | S | $CH_2$(4-t-Bu-Ph) |
| 1-195 | SO | Me |
| 1-196 | SO | Et |
| 1-197 | SO | n-Pr |
| 1-198 | SO | n-Bu |
| 1-199 | SO | t-Bu |
| 1-200 | SO | $CH_2CH=CH_2$ |
| 1-201 | SO | Ph |
| 1-202 | SO | 2-Cl-Ph |
| 1-203 | SO | 3-Cl-Ph |
| 1-204 | SO | 4-Cl-Ph |
| 1-205 | SO | 2-Me-Ph |
| 1-206 | SO | 3-Me-Ph |
| 1-207 | SO | 4-Me-Ph |
| 1-208 | SO | 2-$CO_2$Me-Ph |
| 1-209 | SO | 3-$CO_2$Me-Ph |
| 1-210 | SO | 4-$CO_2$Me-Ph |
| 1-211 | $SO_2$ | Me |
| 1-212 | $SO_2$ | Et |
| 1-213 | $SO_2$ | n-Pr |
| 1-214 | $SO_2$ | n-Bu |
| 1-215 | $SO_2$ | t-Bu |
| 1-216 | $SO_2$ | $CH_2CH=CH_2$ |
| 1-217 | $SO_2$ | Ph |
| 1-218 | $SO_2$ | 2-Cl-Ph |
| 1-219 | $SO_2$ | 3-Cl-Ph |
| 1-220 | $SO_2$ | 4-Cl-Ph |
| 1-221 | $SO_2$ | 2-Me-Ph |
| 1-222 | $SO_2$ | 3-Me-Ph |
| 1-223 | $SO_2$ | 4-Me-Ph |
| 1-224 | $SO_2$ | 2-$CO_2$Me-Ph |
| 1-225 | $SO_2$ | 3-$CO_2$Me-Ph |
| 1-226 | $SO_2$ | 4-$CO_2$Me-Ph |
| 1-227 | $SO_2$ | $CH_2Ph$ |
| 1-228 | $SO_2$ | $CH_2$(4-Cl-Ph) |
| 1-229 | $SO_2$ | $CH_2$(4-t-Bu-Ph) |

Table 1 continued

| Compound No. | Q a | D |
|---|---|---|
| 1-230 | SCO | Me |
| 1-231 | SCO | Et |
| 1-232 | SCO | n-Pr |
| 1-233 | SCO | n-Bu |
| 1-234 | SCO | t-Bu |
| 1-235 | SCO | $CF_3$ |
| 1-236 | SCO | $CH_2Cl$ |
| 1-237 | SCO | $CHCl_2$ |
| 1-238 | SCO | $CCl_3$ |
| 1-239 | SCO | cyc-Pr |
| 1-240 | SCO | cyc-Pen |
| 1-241 | SCO | cyc-Hex |
| 1-242 | SCO | Ph |
| 1-243 | SCO | 2-Cl-Ph |
| 1-244 | SCO | 3-Cl-Ph |
| 1-245 | SCO | 4-Cl-Ph |
| 1-246 | SCO | 2-Me-Ph |
| 1-247 | SCO | 3-Me-Ph |
| 1-248 | SCO | 4-Me-Ph |
| 1-249 | SCO | 2-$CO_2$Me-Ph |
| 1-250 | SCO | 3-$CO_2$Me-Ph |
| 1-251 | SCO | 4-$CO_2$Me-Ph |
| 1-252 | SCO | $CH_2$Ph |
| 1-253 | SCO | $CH_2$(4-Cl-Ph) |
| 1-254 | $SCO_2$ | Me |
| 1-255 | $SCO_2$ | Et |
| 1-256 | $SCO_2$ | n-Pr |
| 1-257 | $SCO_2$ | n-Bu |
| 1-258 | $SCO_2$ | $CH_2CH=CH_2$ |
| 1-259 | $SCO_2$ | Ph |
| 1-260 | $SCO_2$ | $CH_2$Ph |
| 1-261 | SCO | $NH_2$ |
| 1-262 | SCO | NHMe |
| 1-263 | SCO | NHEt |
| 1-264 | SCO | NH(n-Pr) |
| 1-265 | SCO | NH(n-Bu) |
| 1-266 | SCO | $NMe_2$ |
| 1-267 | SCO | $NEt_2$ |

Table 1 continued

| Compound No. | Q a | D |
|---|---|---|
| 1-268 | SCO | NHPh |
| 1-269 | SCO | NH(4-Cl-Ph) |
| 1-270 | SCO | NH(4-Me-Ph) |
| 1-271 | SCO | NH($CH_2$Ph) |
| 1-272 | SCO | NHCH(Me)Ph |
| 1-273 | SCO | N(Me) (Ph) |
| 1-274 | SCO | T1 |
| 1-275 | SCO | T2 |
| 1-276 | $SO_2$ | $NH_2$ |
| 1-277 | $SO_2$ | NHMe |
| 1-278 | $SO_2$ | NHEt |
| 1-279 | $SO_2$ | NH(n-Pr) |
| 1-280 | $SO_2$ | NH(n-Bu) |
| 1-281 | $SO_2$ | NH(t-Bu) |
| 1-282 | $SO_2$ | NHPh |
| 1-283 | $SO_2$ | NH(2-Cl-Ph) |
| 1-284 | $SO_2$ | NH(3-Cl-Ph) |
| 1-285 | $SO_2$ | NH(4-Cl-Ph) |
| 1-286 | $SO_2$ | NH(2-Me-Ph) |
| 1-287 | $SO_2$ | NH(3-Me-Ph) |
| 1-288 | $SO_2$ | NH(4-Me-Ph) |
| 1-289 | $SO_2$ | NH($CH_2$Ph) |
| 1-290 | $SO_2$ | $NHCH_2$(4-Cl-Ph) |
| 1-291 | $SO_2$ | $NMe_2$ |
| 1-292 | $SO_2$ | $NEt_2$ |
| 1-293 | $SO_2$ | N(Me) (Et) |
| 1-294 | $SO_2$ | N(Me) (Ph) |
| 1-295 | $SO_2$ | T1 |
| 1-296 | $SO_2$ | T2 |
| 1-297 | $SO_2$ | T7 |
| 1-298 | $SO_2$ | $N(CH_2CH=CH_2)_2$ |
| 1-299 | $SO_2$ | N(Me) ($CH_2$Ph) |
| 1-300 | $SCH_2$ | $CO_2H$ |
| 1-301 | $SCH_2$ | $CO_2Me$ |
| 1-302 | $SCH_2$ | $CO_2Et$ |
| 1-303 | $SCH_2$ | $CO_2$(n-Pr) |
| 1-304 | $SCH_2$ | $CO_2$(n-Bu) |
| 1-305 | $SCH_2$ | $CO_2Ph$ |

Table 1 continued

| Compound No. | Q a | D |
|---|---|---|
| 1-306 | $SCH_2$ | $CO_2CH_2Ph$ |
| 1-307 | $SCH_2$ | CONHMe |
| 1-308 | $SCH_2$ | CONHEt |
| 1-309 | $SCH_2$ | CONH(n-Pr) |
| 1-310 | $SCH_2$ | CONH(n-Bu) |
| 1-311 | $SCH_2$ | CONHPh |
| 1-312 | $SCH_2$ | $CONH(CH_2Ph)$ |
| 1-313 | $SCH_2$ | $CONMe_2$ |
| 1-314 | $SCH_2$ | $CONEt_2$ |
| 1-315 | $SCH_2$ | CON(Me)(Et) |
| 1-316 | $SCH_2$ | CON(Me)(Ph) |
| 1-317 | $SCH_2$ | T4 |
| 1-318 | $SCH_2$ | T5 |
| 1-319 | $SCH_2$ | T6 |
| 1-320 | SCH(Me) | $CO_2Me$ |
| 1-321 | SCH(Me) | $CO_2Et$ |
| 1-322 | SCH(Me) | $CO_2(n-Pr)$ |
| 1-323 | SCH(Me) | $CO_2(n-Bu)$ |
| 1-324 | SCH(Me) | $CO_2Ph$ |
| 1-325 | SCH(Me) | $CO_2CH_2Ph$ |
| 1-326 | SCH(Me) | CONHMe |
| 1-327 | SCH(Me) | CONHEt |
| 1-328 | SCH(Me) | CONHPh |
| 1-329 | SCH(Me) | $CONH(CH_2Ph)$ |
| 1-330 | SCH(Me) | $CONMe_2$ |
| 1-331 | SCH(Me) | $CONEt_2$ |
| 1-332 | O | $CH_2CF_3$ |
| 1-333 | O | $CH_2CH_2CH_2Cl$ |
| 1-334 | $OC(Me)_2$ | $CO_2Me$ |
| 1-335 | $OC(Me)_2$ | $CO_2Et$ |
| 1-336 | $OC(Me)_2$ | $CO_2(n-Pr)$ |
| 1-337 | $OC(Me)_2$ | $CO_2(n-Bu)$ |
| 1-338 | $OC(Me)_2$ | CONHMe |
| 1-339 | $OC(Me)_2$ | CONHEt |
| 1-340 | $OC(Me)_2$ | $CONMe_2$ |
| 1-341 | $OC(Me)_2$ | $CONEt_2$ |
| 1-342 | $SC(Me)_2$ | $CO_2Me$ |
| 1-343 | $SC(Me)_2$ | $CO_2Et$ |

Table 1 continued

| Compound No. | Q a | D |
|---|---|---|
| 1-344 | $SC(Me)_2$ | $CO_2$(n-Pr) |
| 1-345 | $SC(Me)_2$ | $CO_2$(n-Bu) |
| 1-346 | $SC(Me)_2$ | CONHMe |
| 1-347 | $SC(Me)_2$ | CONHEt |
| 1-348 | $SC(Me)_2$ | $CONMe_2$ |
| 1-349 | $SC(Me)_2$ | $CONEt_2$ |

In Table 1, T1 to T7 denote following structures.

T1 ; 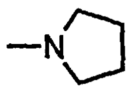

T5 ; 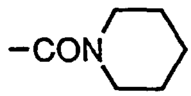

T2 ; 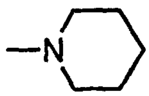

T6 ; 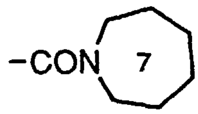

T3 ; 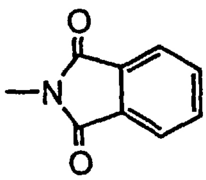

T7 ; 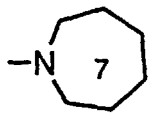

T4 ; 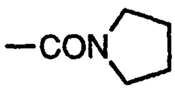

Table 2

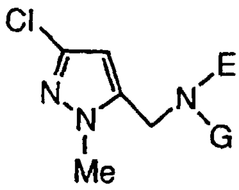 ,

(a)

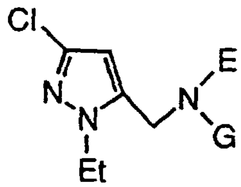 ,

(b)

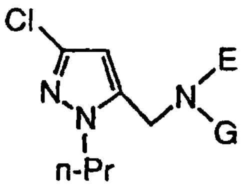 ,

(c)

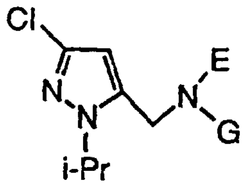 ,

(d)

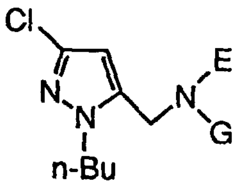 ,

(e)

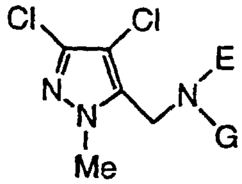 ,

(f)

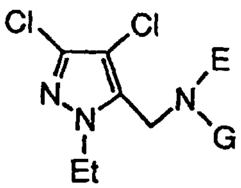 ,

(g)

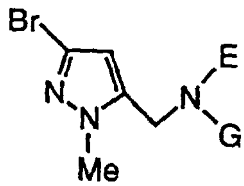 ,

(h)

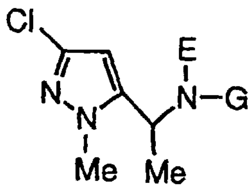

(i)

or

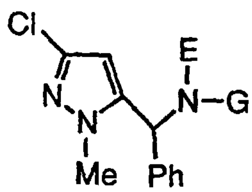

(j)

.

| Compound No. | E | G |
|---|---|---|
| 2-1 | H | H |
| 2-2 | H | Me |
| 2-3 | H | Et |
| 2-4 | H | n-Pr |
| 2-5 | H | i-Pr |
| 2-6 | H | n-Bu |
| 2-7 | H | s-Bu |
| 2-8 | H | t-Bu |
| 2-9 | H | n-Pen |
| 2-10 | H | n-Hex |
| 2-11 | H | n-Hep |
| 2-12 | H | n-Oct |
| 2-13 | H | n-Non |
| 2-14 | H | n-Dec |
| 2-15 | H | n-Dod |
| 2-16 | H | i-Bu |
| 2-17 | H | cyc-Pr |
| 2-18 | H | cyc-Pen |
| 2-19 | H | cyc-Hex |
| 2-20 | H | cyc-Hep |
| 2-21 | H | $CH=CH_2$ |
| 2-22 | H | $CH_2CH=CH_2$ |
| 2-23 | H | $C(Me)_2CH=CH_2$ |
| 2-24 | H | $CH_2CH=CHMe$ |
| 2-25 | H | $CH_2C(Me)=CH_2$ |
| 2-26 | H | Ph |
| 2-27 | H | 2-Cl-Ph |
| 2-28 | H | 3-Cl-Ph |
| 2-29 | H | 4-Cl-Ph |
| 2-30 | H | 2-Me-Ph |
| 2-31 | H | 3-Me-Ph |
| 2-32 | H | 4-Me-Ph |
| 2-33 | H | 2-CN-Ph |
| 2-34 | H | 3-CN-Ph |
| 2-35 | H | 4-CN-Ph |
| 2-36 | H | 2-$CO_2H$-Ph |
| 2-37 | H | 3-$CO_2H$-Ph |
| 2-38 | H | 4-$CO_2H$-Ph |
| 2-39 | H | 2-$CO_2Me$-Ph |

Table 2 continued

| Compound No. | E | G |
|---|---|---|
| 2-40 | H | 3-$CO_2$Me-Ph |
| 2-41 | H | 4-$CO_2$Me-Ph |
| 2-42 | H | 2-$NO_2$-Ph |
| 2-43 | H | 3-$NO_2$-Ph |
| 2-44 | H | 4-$NO_2$-Ph |
| 2-45 | H | 4-$NH_2$-Ph |
| 2-46 | H | 2-$CF_3$-Ph |
| 2-47 | H | 3-$CF_3$-Ph |
| 2-48 | H | 4-$CF_3$-Ph |
| 2-49 | H | 4-HO-Ph |
| 2-50 | H | 1-Nap |
| 2-51 | H | 2-Nap |
| 2-52 | H | $CH_2$Ph |
| 2-53 | H | $CH_2$(4-Me-Ph) |
| 2-54 | H | $CH_2$(4-Cl-Ph) |
| 2-55 | H | $CH_2$(4-CN-Ph) |
| 2-56 | H | $CH_2$(4-$NO_2$-Ph) |
| 2-57 | H | $CH_2$(1-Nap) |
| 2-58 | H | $CH_2$(2-Nap) |
| 2-59 | H | CH(Me)Ph |
| 2-60 | H | CH(Me)(4-Cl-Ph) |
| 2-61 | H | CH(Me)(4-Me-Ph) |
| 2-62 | H | CH(Me)(2,4-$Cl_2$-Ph) |
| 2-63 | H | $CH_2CH_2$Ph |
| 2-64 | Me | Me |
| 2-65 | Me | Et |
| 2-66 | Me | n-Pr |
| 2-67 | Me | n-Bu |
| 2-68 | Me | Ph |
| 2-69 | Me | 4-Cl-Ph |
| 2-70 | Me | 4-Me-Ph |
| 2-71 | Me | $CH_2$Ph |
| 2-72 | Me | $CH_2$(4-Cl-Ph) |
| 2-73 | Me | $CH_2$(1-Nap) |
| 2-74 | Me | $CH_2$(2-Nap) |
| 2-75 | Me | CH(Me)Ph |
| 2-76 | Me | CH(Me)(4-Cl-Ph) |
| 2-77 | Ph | Ph |

Table 2 continued

| Compound No. | E | G |
|---|---|---|
| 2-78 | Ph | $CH_2Ph$ |
| 2-79 | H | COMe |
| 2-80 | H | COEt |
| 2-81 | H | CO(n-Pr) |
| 2-82 | H | CO(n-Bu) |
| 2-83 | H | $COCH_2CH=CH_2$ |
| 2-84 | H | CO-cyc-Pr |
| 2-85 | H | CO-cyc-Pen |
| 2-86 | H | CO-cyc-Hex |
| 2-87 | H | $COCF_3$ |
| 2-88 | H | $COCH_2Cl$ |
| 2-89 | H | $COCHCl_2$ |
| 2-90 | H | $COCCl_3$ |
| 2-91 | H | COPh |
| 2-92 | H | CO(2-Me-Ph) |
| 2-93 | H | CO(3-Me-Ph) |
| 2-94 | H | CO(4-Me-Ph) |
| 2-95 | H | CO(2-Cl-Ph) |
| 2-96 | H | CO(3-Cl-Ph) |
| 2-97 | H | CO(4-Cl-Ph) |
| 2-98 | H | CO(2-$CF_3$-Ph) |
| 2-99 | H | CO(3-$CF_3$-Ph) |
| 2-100 | H | CO(4-$CF_3$-Ph) |
| 2-101 | H | CO(2-CN-Ph) |
| 2-102 | H | CO(3-CN-Ph) |
| 2-103 | H | CO(4-CN-Ph) |
| 2-104 | H | CO(2-$CO_2H$-Ph) |
| 2-105 | H | CO(3-$CO_2H$-Ph) |
| 2-106 | H | CO(4-$CO_2H$-Ph) |
| 2-107 | H | CO(2-$CO_2Me$-Ph) |
| 2-108 | H | CO(3-$CO_2Me$-Ph) |
| 2-109 | H | CO(4-$CO_2Me$-Ph) |
| 2-110 | H | CO(2-HO-Ph) |
| 2-111 | H | CO(2-$NH_2$-Ph) |
| 2-112 | H | $COCH_2Ph$ |
| 2-113 | H | $COCH_2$(2-Cl-Ph) |
| 2-114 | H | $COCH_2$(3-Cl-Ph) |
| 2-115 | H | $COCH_2$(4-Cl-Ph) |

Table 2 continued

| Compound No. | E | G |
|---|---|---|
| 2-116 | H | $COCH_2$(2-Me-Ph) |
| 2-117 | H | $COCH_2$(3-Me-Ph) |
| 2-118 | H | $COCH_2$(4-Me-Ph) |
| 2-119 | H | $CO_2$(t-Bu) |
| 2-120 | H | $CO_2Me$ |
| 2-121 | H | $CO_2Et$ |
| 2-122 | H | $CO_2$(n-Pr) |
| 2-123 | H | $CO_2$(n-Bu) |
| 2-124 | H | $CO_2Ph$ |
| 2-125 | H | $CO_2CH_2Ph$ |
| 2-126 | H | CONHMe |
| 2-127 | H | CONHEt |
| 2-128 | H | CONH(n-Pr) |
| 2-129 | H | CONH(n-Bu) |
| 2-130 | H | CONHPh |
| 2-131 | H | CONH(4-Cl-Ph) |
| 2-132 | H | CONH(4-Me-Ph) |
| 2-133 | H | CONH($CH_2Ph$) |
| 2-134 | H | CONHCH(Me)Ph |
| 2-135 | H | $CONMe_2$ |
| 2-136 | H | $CONEt_2$ |
| 2-137 | H | CON(Me)(Ph) |
| 2-138 | H | CO-T1 |
| 2-139 | H | CO-T2 |
| 2-140 | H | CO-T7 |
| 2-141 | H | $CH_2CO_2H$ |
| 2-142 | H | $CH_2CO_2Me$ |
| 2-143 | H | $CH_2CO_2Et$ |
| 2-144 | H | $CH_2CO_2$(n-Pr) |
| 2-145 | H | $CH_2CO_2$(n-Bu) |
| 2-146 | H | $CH_2CO_2CH_2Ph$ |
| 2-147 | H | CH(Me)$CO_2H$ |
| 2-148 | H | CH(Me)$CO_2Me$ |
| 2-149 | H | CH(Me)$CO_2Et$ |
| 2-150 | H | CH(Me)$CO_2$(n-Pr) |
| 2-151 | H | CH(Me)$CO_2$(n-Bu) |
| 2-152 | H | CH(Me)$CO_2CH_2Ph$ |
| 2-153 | H | CH(i-Pr)$CO_2H$ |

Table 2 continued

| Compound No. | E | G |
|---|---|---|
| 2-154 | H | $CH(i-Pr)CO_2Me$ |
| 2-155 | H | $CH(i-Pr)CO_2Et$ |
| 2-156 | H | $CH(i-Pr)CO_2(n-Pr)$ |
| 2-157 | H | $CH(i-Pr)CO_2(n-Bu)$ |
| 2-158 | H | $CH(i-Pr)CO_2CH_2Ph$ |
| 2-159 | H | $CH(CH_2CH_2SMe)CO_2H$ |
| 2-160 | H | $CH(CH_2CH_2SMe)CO_2Me$ |
| 2-161 | H | $CH(CH_2CH_2SMe)CO_2Et$ |
| 2-162 | H | $CH(CH_2CH_2SMe)CO_2(n-Pr)$ |
| 2-163 | H | $CH(CH_2CH_2SMe)CO_2(n-Bu)$ |
| 2-164 | H | $CH(CH_2CH_2SMe)CO_2CH_2Ph$ |
| 2-165 | H | $CH(CH_2Ph)CO_2H$ |
| 2-166 | H | $CH(CH_2Ph)CO_2Me$ |
| 2-167 | H | $CH(CH_2Ph)CO_2Et$ |
| 2-168 | H | $CH(CH_2Ph)CO_2(n-Pr)$ |
| 2-169 | H | $CH(CH_2Ph)CO_2(n-Bu)$ |
| 2-170 | H | $CH(CH_2Ph)CO_2CH_2Ph$ |
| 2-171 | H | $C(Me)_2CO_2H$ |
| 2-172 | H | $C(Me)_2CO_2Me$ |
| 2-173 | H | $C(Me)_2CO_2Et$ |
| 2-174 | H | $C(Me)_2CO_2(n-Pr)$ |
| 2-175 | H | $C(Me)_2CO_2(n-Bu)$ |
| 2-176 | H | $C(Me)_2CO_2CH_2Ph$ |
| 2-177 | Me | $CH_2CO_2H$ |
| 2-178 | Me | $CH_2CO_2Me$ |
| 2-179 | Me | $CH_2CO_2Et$ |
| 2-180 | Me | $CH_2CO_2(n-Pr)$ |
| 2-181 | Me | $CH_2CO_2(n-Bu)$ |
| 2-182 | Me | $CH_2CO_2CH_2Ph$ |
| 2-183 | Et | Et |
| 2-184 | Et | n-Pr |
| 2-185 | Et | n-Bu |
| 2-186 | Et | Ph |
| 2-187 | Et | $CH_2Ph$ |
| 2-188 | n-Pr | n-Pr |
| 2-189 | n-Pr | n-Bu |
| 2-190 | n-Pr | Ph |
| 2-191 | n-Pr | $CH_2Ph$ |

Table 2 continued

| Compound No. | E | G |
|---|---|---|
| 2-192 | i-Pr | Me |
| 2-193 | i-Pr | Et |
| 2-194 | i-Pr | n-Pr |
| 2-195 | i-Pr | i-Pr |
| 2-196 | i-Pr | n-Bu |
| 2-197 | i-Pr | Ph |
| 2-198 | i-Pr | $CH_2Ph$ |
| 2-199 | n-Bu | n-Bu |
| 2-200 | n-Bu | Ph |
| 2-201 | n-Bu | $CH_2Ph$ |
| 2-202 | $CH_2Ph$ | $CH_2Ph$ |
| 2-203 | $CH_2CH_2CH_2$ | |
| 2-204 | $CH_2CH_2CH_2CH_2$ | |
| 2-205 | $CH_2CH_2CH_2CH_2CH_2$ | |
| 2-206 | $CH_2CH_2CH_2CH_2CH_2CH_2$ | |
| 2-207 | $CH_2CH_2OCH_2CH_2$ | |
| 2-208 | $CH_2CH_2SCH_2CH_2$ | |
| 2-209 | $CH_2CH(Me)OCH(Me)CH_2$ | |
| 2-210 | $CH_2CH_2NHCH_2CH_2$ | |
| 2-211 | $CH_2CH_2N(Me)CH_2CH_2$ | |
| 2-212 | $CH_2CH_2N(Et)CH_2CH_2$ | |
| 2-213 | $CH_2CH_2N(CH_2Ph)CH_2CH_2$ | |
| 2-214 | $COCH_2CH_2CO$ | |
| 2-215 | $COCH(Me)CH_2CO$ | |
| 2-216 | $COC(=CH_2)CH_2CO$ | |
| 2-217 | $COCH_2CH_2CH_2CO$ | |
| 2-218 | $COCH_2CH_2CH_2CH_2CO$ | |
| 2-219 | $COCH_2CH_2SO_2$ | |
| 2-220 | $CH(CO_2Me)CH_2CH_2CH_2$ | |
| 2-221 | $CH(CO_2Et)CH_2CH_2CH_2$ | |
| 2-222 | COCH=CHCO | |
| 2-223 | COC(Me)=C(Me)CO | |
| 2-224 | COC(Cl)=C(Cl)CO | |
| 2-225 | H | $SO_2Me$ |
| 2-226 | H | $SO_2Et$ |
| 2-227 | H | $SO_2$(n-Pr) |
| 2-228 | H | $SO_2$(n-Bu) |
| 2-229 | H | $SO_2$(n-Pen) |

Table 2 continued

| Compound No. | E | G |
|---|---|---|
| 2-230 | H | $SO_2$(n-Hex) |
| 2-231 | H | $SO_2Ph$ |
| 2-232 | H | $SO_2$(4-Me-Ph) |
| 2-233 | H | $SO_2$(4-Cl-Ph) |
| 2-234 | H | $SO_2CH_2Ph$ |
| 2-235 | H | $SO_2CH_2$(4-Me-Ph) |
| 2-236 | H | $SO_2CH_2$(4-Cl-Ph) |
| 2-237 | H | $SO_2$-cyc-Pr |
| 2-238 | H | $SO_2$-cyc-Pen |
| 2-239 | H | $SO_2$-cyc-Hex |
| 2-240 | H | $SO_2CH_2CO_2Me$ |
| 2-241 | H | $SO_2CH_2CO_2Et$ |
| 2-242 | H | OH |
| 2-243 | H | OMe |
| 2-244 | H | OEt |
| 2-245 | H | O(i-Pr) |
| 2-246 | H | O(n-Bu) |
| 2-247 | H | O(n-Pen) |
| 2-248 | H | O(n-Hex) |
| 2-249 | H | O(n-Hep) |
| 2-250 | H | O(n-Oct) |
| 2-251 | H | O(n-Non) |
| 2-252 | H | O(n-Dec) |
| 2-253 | H | O-cyc-Pr |
| 2-254 | H | O-cyc-Pen |
| 2-255 | H | O-cyc-Hex |
| 2-256 | H | $OCH_2CH=CH_2$ |
| 2-257 | H | $OCH_2C(Me)=CH_2$ |
| 2-258 | H | $OCH_2CH=CHCl$ |
| 2-259 | H | $OCH_2C \equiv CH$ |
| 2-260 | H | $OCH_2Ph$ |
| 2-261 | H | $OCH_2$(4-Me-Ph) |
| 2-262 | H | $OCH_2$(4-Cl-Ph) |
| 2-263 | H | $OCH_2CO_2Me$ |
| 2-264 | H | $OCH_2CO_2Et$ |
| 2-265 | H | $OCH_2CO_2$(n-Pr) |
| 2-266 | Me | OH |
| 2-267 | Me | OMe |

Table 2 continued

| Compound No. | E | G |
|---|---|---|
| 2-268 | Me | OEt |
| 2-269 | Me | $OCH_2Ph$ |
| 2-270 | Me | COMe |
| 2-271 | Me | COEt |
| 2-272 | Me | COPh |
| 2-273 | Me | $COCH_2Ph$ |
| 2-274 | Me | CONHMe |
| 2-275 | Me | CONHEt |
| 2-276 | Me | CONHPh |
| 2-277 | Me | $CONH(CH_2Ph)$ |
| 2-278 | Et | COMe |
| 2-279 | Et | COEt |
| 2-280 | Et | COPh |
| 2-281 | Et | $COCH_2Ph$ |
| 2-282 | Et | CONHMe |
| 2-283 | Et | CONHEt |
| 2-284 | Et | CONHPh |
| 2-285 | Et | $CONH(CH_2Ph)$ |
| 2-286 | n-Pr | COMe |
| 2-287 | n-Pr | COEt |
| 2-288 | n-Pr | COPh |
| 2-289 | n-Pr | $COCH_2Ph$ |
| 2-290 | n-Pr | CONHMe |
| 2-291 | n-Pr | CONHEt |
| 2-292 | n-Pr | CONHPh |
| 2-293 | n-Pr | $CONH(CH_2Ph)$ |
| 2-294 | n-Bu | COMe |
| 2-295 | n-Bu | COEt |
| 2-296 | n-Bu | COPh |
| 2-297 | n-Bu | $COCH_2Ph$ |
| 2-298 | n-Bu | CONHMe |
| 2-299 | n-Bu | CONHEt |
| 2-300 | n-Bu | CONHPh |
| 2-301 | n-Bu | $CONH(CH_2Ph)$ |
| 2-302 | cyc-Pen | COMe |
| 2-303 | cyc-Pen | COEt |
| 2-304 | cyc-Pen | COPh |
| 2-305 | cyc-Pen | $COCH_2Ph$ |

Table 2 continued

| Compound No. | E | G |
|---|---|---|
| 2-306 | cyc-Pen | CONHMe |
| 2-307 | cyc-Pen | CONHEt |
| 2-308 | cyc-Pen | CONHPh |
| 2-309 | cyc-Pen | CONH($CH_2$Ph) |
| 2-310 | cyc-Hex | COMe |
| 2-311 | cyc-Hex | COEt |
| 2-312 | cyc-Hex | COPh |
| 2-313 | cyc-Hex | $COCH_2$Ph |
| 2-314 | cyc-Hex | CONHMe |
| 2-315 | cyc-Hex | CONHEt |
| 2-316 | cyc-Hex | CONHPh |
| 2-317 | cyc-Hex | CONH($CH_2$Ph) |
| 2-318 | Me | $CONMe_2$ |
| 2-319 | Me | $CONEt_2$ |
| 2-320 | Me | CON(Me)(Ph) |
| 2-321 | Et | $CONMe_2$ |
| 2-322 | Et | $CONEt_2$ |
| 2-323 | Et | CON(Me)(Ph) |
| 2-324 | n-Pr | $CONMe_2$ |
| 2-325 | n-Pr | $CONEt_2$ |
| 2-326 | n-Pr | CON(Me)(Ph) |
| 2-327 | n-Bu | $CONMe_2$ |
| 2-328 | n-Bu | $CONEt_2$ |
| 2-329 | n-Bu | CON(Me)(Ph) |
| 2-330 | cyc-Pen | $CONMe_2$ |
| 2-331 | cyc-Pen | $CONEt_2$ |
| 2-332 | cyc-Pen | CON(Me)(Ph) |
| 2-333 | cyc-Hex | $CONMe_2$ |
| 2-334 | cyc-Hex | $CONEt_2$ |
| 2-335 | cyc-Hex | CON(Me)(Ph) |
| 2-336 | H | CSMe |
| 2-337 | H | CSEt |
| 2-338 | H | CS(n-Pr) |
| 2-339 | H | CS(n-Bu) |
| 2-340 | H | CSPh |
| 2-341 | H | $CSCH_2$Ph |
| 2-342 | H | CSNHMe |
| 2-343 | H | CSNHEt |

Table 2 continued

| Compound No. | E | G |
|---|---|---|
| 2-344 | H | CSNH(n-Pr) |
| 2-345 | H | CHNH(n-Bu) |
| 2-346 | H | CSNHPh |
| 2-347 | H | CSNH(4-Cl-Ph) |
| 2-348 | H | CSNH(4-Me-Ph) |
| 2-349 | H | $CSNHCH_2Ph$ |
| 2-350 | H | CSNHCH(Me)Ph |
| 2-351 | Me | CSNHMe |
| 2-352 | Me | CSNHEt |
| 2-353 | Me | CSNH(n-Pr) |
| 2-354 | Me | CSNH(n-Bu) |
| 2-355 | Me | CSNHPh |
| 2-356 | Me | CSNH(4-Cl-Ph) |
| 2-357 | Me | CSNH(4-Me-Ph) |
| 2-358 | Me | $CSNHCH_2Ph$ |
| 2-359 | Me | CSNHCH(Me)Ph |
| 2-360 | Et | CSNHMe |
| 2-361 | Et | CSNHEt |
| 2-362 | Et | CSNH(n-Pr) |
| 2-363 | Et | CSNH(n-Bu) |
| 2-364 | Et | CSNHPh |
| 2-365 | Et | CSNH(4-Cl-Ph) |
| 2-366 | Et | CSNH(4-Me-Ph) |
| 2-367 | Et | $CSNH(CH_2Ph)$ |
| 2-368 | Et | CSNHCH(Me)Ph |
| 2-369 | n-Pr | CSNHMe |
| 2-370 | n-Pr | CSNHPh |
| 2-371 | n-Bu | CSNHMe |
| 2-372 | n-Bu | CSNHPh |
| 2-373 | cyc-Pen | CSNHMe |
| 2-374 | cyc-Pen | CSNHPh |
| 2-375 | cyc-Hex | CSNHMe |
| 2-376 | cyc-Hex | CSNHPh |
| 2-377 | Ph | CSNHMe |
| 2-378 | Ph | CSNHEt |
| 2-379 | Ph | CSNH(n-Pr) |
| 2-380 | Ph | CSNH(n-Bu) |
| 2-381 | Ph | CSNHPh |

Table 2 continued

| Compound No. | E | G |
|---|---|---|
| 2-382 | Ph | $CSNH(CH_2Ph)$ |
| 2-383 | H | $CH_2CH_2(4\text{-}Cl\text{-}Ph)$ |
| 2-384 | H | $CH_2CH_2(4\text{-}Me\text{-}Ph)$ |
| 2-385 | H | $CH_2CH_2(4\text{-}MeO\text{-}Ph)$ |
| 2-386 | H | $CH_2CH_2(3,4\text{-}(MeO)_2\text{-}Ph)$ |
| 2-387 | Me | $CH_2CH_2Ph$ |
| 2-388 | Me | $CH_2CH_2(4\text{-}Cl\text{-}Ph)$ |
| 2-389 | Me | $CH_2CH_2(3,4\text{-}(MeO)_2\text{-}Ph)$ |
| 2-390 | Et | $CH_2(4\text{-}Cl\text{-}Ph)$ |
| 2-391 | Et | $CH_2CH_2Ph$ |
| 2-392 | Et | $CH_2CH_2(4\text{-}Cl\text{-}Ph)$ |
| 2-393 | n-Pr | $CH_2CH_2Ph$ |
| 2-394 | n-Pr | $CH_2CH_2(4\text{-}Cl\text{-}Ph)$ |
| 2-395 | i-Pr | $CH_2CH_2Ph$ |
| 2-396 | n-Bu | $CH_2CH_2Ph$ |
| 2-397 | H | $CH_2CH_2CH_2Cl$ |
| 2-398 | H | $NH_2$ |
| 2-399 | H | NHMe |
| 2-400 | H | N(Me)(Ph) |
| 2-401 | H | $NMe_2$ |
| 2-402 | H | NH(t-Bu) |
| 2-403 | H | NHPh |
| 2-404 | H | $NH(CH_2Ph)$ |
| 2-405 | H | $NPh_2$ |
| 2-406 | Me | $NH_2$ |
| 2-407 | Me | NHMe |
| 2-408 | Me | NHPh |
| 2-409 | H | $CH_2CH(Et)(CH_2)_3CH_3$ |
| 2-410 | $CH_2CH(Et)(CH_2)_3CH_3$ | $CH_2CH(Et)(CH_2)_3CH_3$ |
| 2-411 | H | $CH_2CH_2OMe$ |
| 2-412 | H | $CH_2CH_2OEt$ |
| 2-413 | i-Bu | i-Bu |
| 2-414 | $CH_2CH=CH_2$ | $CH_2CH=CH_2$ |
| 2-415 | $CH(Me)CH_2CH_2CH_2CH_2$ | |
| 2-416 | $CH_2CH(Me)CH_2CH_2CH_2$ | |
| 2-417 | $CH_2CH_2CH(Me)CH_2CH_2$ | |
| 2-418 | $CH_2CH_2CH_2CH_2CH_2CH_2CH_2$ | |
| 2-419 | $CH_2C(Me)_2CH_2CH(Me)CH_2CH_2$ | |

Table 2 continued

| Compound No. | E | G |
|---|---|---|
| 2-420 | $CH_2CH(Me)NHCH(Me)CH_2$ | |
| 2-421 | H | $CH_2CH_2CN$ |
| 2-422 | $CH_2CH_2CN$ | $CH_2CH_2CN$ |
| 2-423 | H | CO-T8 |
| 2-424 | H | $CH_2CH_2CH_2OMe$ |
| 2-425 | H | $CH_2CH_2CH_2OEt$ |
| 2-426 | H | i-Bu |
| 2-427 | H | $C(Me)_2Ph$ |
| 2-428 | $COCH_2C(Me)_2CH_2CO$ | |
| 2-429 | H | CONH(t-Bu) |
| 2-430 | CHO | $CH(CH_2CH_2SMe)CO_2Et$ |
| 2-431 | $COCH_2CH_2CH_2$ | |
| 2-432 | $COCH_2CH_2CH_2CH_2$ | |
| 2-433 | H | CHO |
| 2-434 | Me | CHO |
| 2-435 | Et | CHO |
| 2-436 | Me | $CH_2CH=CH_2$ |
| 2-437 | Et | $CH_2CH=CH_2$ |
| 2-438 | n-Pr | $CH_2CH=CH_2$ |
| 2-439 | i-Pr | $CH_2CH=CH_2$ |
| 2-440 | n-Bu | $CH_2CH=CH_2$ |
| 2-441 | i-Bu | $CH_2CH=CH_2$ |
| 2-442 | s-Bu | $CH_2CH=CH_2$ |
| 2-443 | cyc-Pr | $CH_2CH=CH_2$ |
| 2-444 | cyc-Pen | $CH_2CH=CH_2$ |
| 2-445 | cyc-Hex | $CH_2CH=CH_2$ |
| 2-446 | Ph | $CH_2CH=CH_2$ |
| 2-447 | 4-Cl-Ph | $CH_2CH=CH_2$ |
| 2-448 | 4-Me-Ph | $CH_2CH=CH_2$ |
| 2-449 | $CH_2Ph$ | $CH_2CH=CH_2$ |
| 2-450 | $CH_2CH=CHCH_2CH_2$ | |
| 2-451 | $CH_2CH_2N(n\text{-}Pr)CH_2CH_2$ | |
| 2-452 | $CH_2CH_2N(n\text{-}Bu)CH_2CH_2$ | |
| 2-453 | $CH_2CH_2N(CH_2CH=CH_2)CH_2CH_2$ | |
| 2-454 | $CH_2CH_2N(COMe)CH_2CH_2$ | |
| 2-455 | $CH_2CH_2N(COEt)CH_2CH_2$ | |
| 2-456 | $CH_2CH_2N(CO_2Me)CH_2CH_2$ | |
| 2-457 | $CH_2CH_2N(CO_2Et)CH_2CH_2$ | |

Table 2 continued

| Compound No. | E | G |
|---|---|---|
| 2-458 | $CH_2CH_2N(Ph)CH_2CH_2$ | |
| 2-459 | $CH_2CH_2N(cyc\text{-}Pen)CH_2CH_2$ | |
| 2-460 | $CH_2CH_2N(cyc\text{-}Hex)CH_2CH_2$ | |
| 2-461 | $CH_2CH(Me)N(Me)CH(Me)CH_2$ | |
| 2-462 | $CH_2CH(Me)N(CH_2CH{=}CH_2)CH(Me)CH_2$ | |
| 2-463 | $CH_2CH(Me)N(COMe)CH(Me)CH_2$ | |
| 2-464 | $CH_2CH(Me)N(CO_2Me)CH(Me)CH_2$ | |
| 2-465 | $CH_2CH(Me)N(CO_2Et)CH(Me)CH_2$ | |
| 2-466 | $CH(Me)CH_2NHCH_2CH_2$ | |
| 2-467 | $CH(Me)CH_2NHCH_2CH(Me)$ | |
| 2-468 | $CH_2CH(Me)NHCH_2CH_2$ | |
| 2-469 | $CH_2CH_2NHCH_2CH_2CH_2$ | |
| 2-470 | $CH(Me)CH_2NHCH(Me)CH_2$ | |
| 2-471 | $CH_2CH(OH)CH_2CH_2CH_2$ | |
| 2-472 | $CH_2CH_2CH(OH)CH_2CH_2$ | |
| 2-473 | H | $CH_2CH_2NHMe$ |
| 2-474 | H | $CH_2CH_2NHEt$ |
| 2-475 | H | $CH_2CH_2N(Me)_2$ |
| 2-476 | H | $CH_2CH_2N(Et)_2$ |
| 2-477 | H | $CH_2CH_2CH_2NHMe$ |
| 2-478 | H | $CH_2CH_2CH_2NHEt$ |
| 2-479 | H | $CH_2CH_2CH_2N(Me)_2$ |
| 2-480 | H | $CH_2CH_2CH_2N(Et)_2$ |
| 2-481 | H | $CH_2CH_2CH_2\text{-}T8$ |
| 2-482 | Me | $CH_2CH_2N(Me)_2$ |
| 2-483 | Me | $CH_2CH_2N(Et)_2$ |
| 2-484 | Me | $CH_2CH_2CH_2N(Me)_2$ |
| 2-485 | Me | $CH_2CH_2CH_2N(Et)_2$ |
| 2-486 | Me | $CH_2CH_2CH_2\text{-}T8$ |
| 2-487 | Et | $CH_2CH_2N(Me)_2$ |
| 2-488 | Et | $CH_2CH_2N(Et)_2$ |
| 2-489 | Et | $CH_2CH_2CH_2N(Me)_2$ |
| 2-490 | Et | $CH_2CH_2CH_2N(Et)_2$ |

In Table 2, T1, T2 17 and T8 denote following structures.

T1 ; 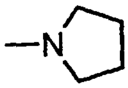

T7 ; 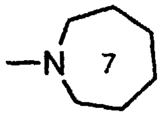

T2 ; 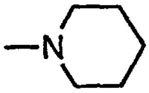

T8 ; 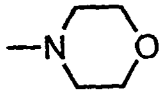

Table 3

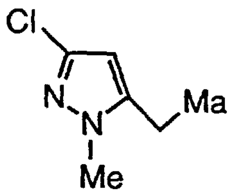,

(a)

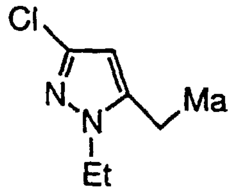,

(b)

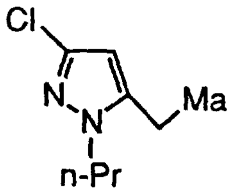,

(c)

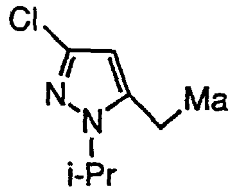,

(d)

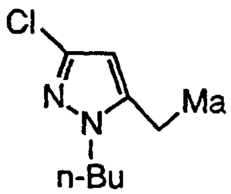,

(e)

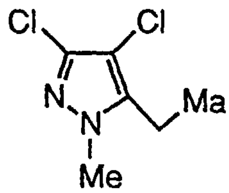,

(f)

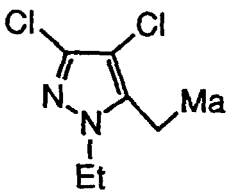,

(g)

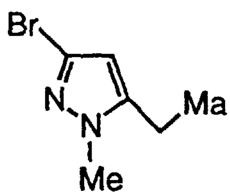,

(h)

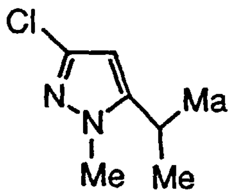

(i)

or

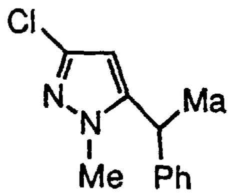

(j)

| Compound No. | Ma |
|---|---|
| 3-1 | $N_3$ |
| 3-2 | V1 |
| 3-3 | V2 |
| 3-4 | V3 |
| 3-5 | V4 |
| 3-6 | V5 |
| 3-7 | V6 |
| 3-8 | V7 |
| 3-9 | V8 |
| 3-10 | V9 |
| 3-11 | V10 |
| 3-12 | V11 |
| 3-13 | V12 |
| 3-14 | V13 |
| 3-15 | V14 |
| 3-16 | V15 |
| 3-17 | V16 |
| 3-18 | V17 |
| 3-19 | V18 |
| 3-20 | V19 |
| 3-21 | V20 |
| 3-22 | V21 |
| 3-23 | V22 |
| 3-24 | V23 |
| 3-25 | V24 |
| 3-26 | V25 |
| 3-27 | V26 |
| 3-28 | V27 |
| 3-29 | V28 |
| 3-30 | V29 |

In Table 3, V1 to V29 denote following structures.

V1 ; 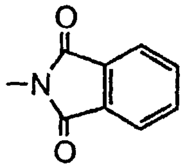

V2 ; 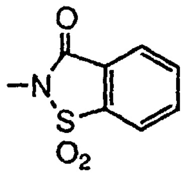

V3 ; 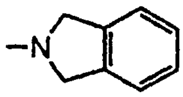

V4 ; 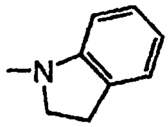

V5 ; 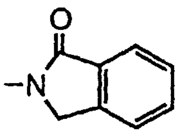

V6 ; 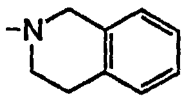

V7 ; 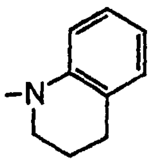

V8 ; 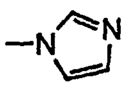

V9 ; 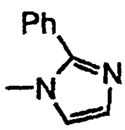

V10 ; 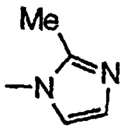

V11 ; 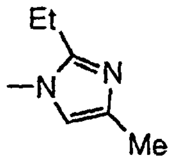

V12 ; 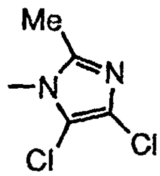

V13 ; 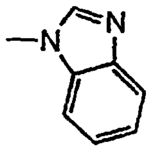

V14 ; 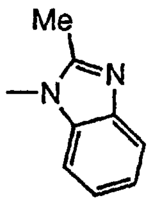

V15 ; 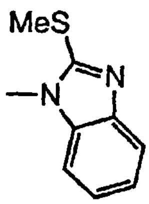

V16 ; 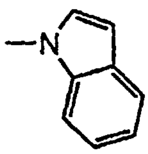

V17 ; 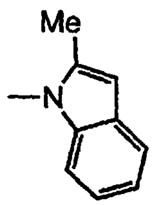

V18 ; 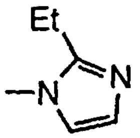

V19 ; 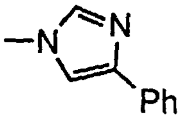

V20 ; 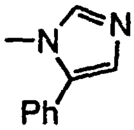

V21 ; 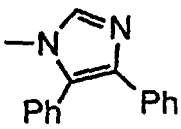

V22 ; 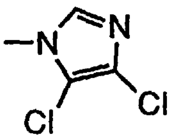

V23 ; 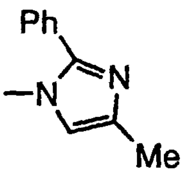

V24 ; 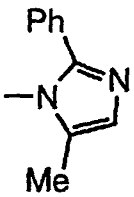

V25 ; 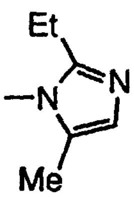

V26 ; 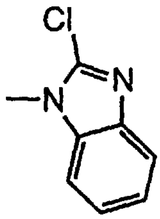

V27 ; 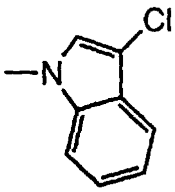

V28 ; 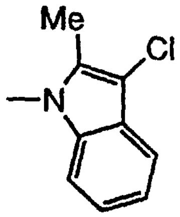

V29 ; 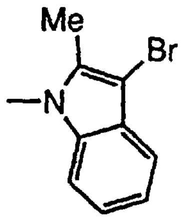

## Table 4

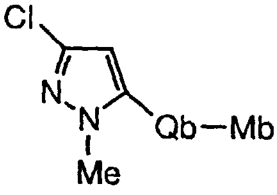
,

(a)

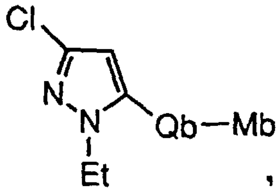
,

(b)

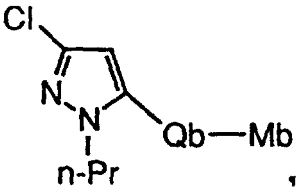
,

(c)

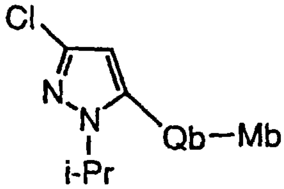
,

(d)

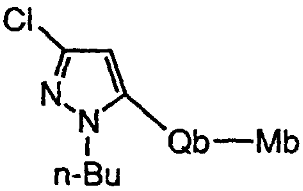
,

(e)

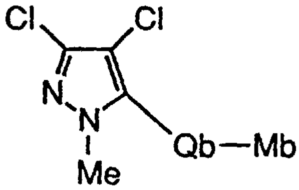
,

(f)

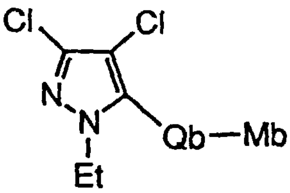

(g)

or

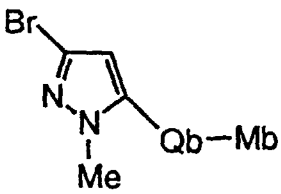

(h)

| Compound No. | Q b | M b |
|---|---|---|
| 4-1 | CH=CH | CHO |
| 4-2 | CH=CH | CN |
| 4-3 | CH=CH | $CO_2Me$ |
| 4-4 | CH=CH | $CO_2Et$ |
| 4-5 | CH=CH | COMe |
| 4-6 | CH=CH | COEt |
| 4-7 | CH=CH | COPh |
| 4-8 | CH=CH | $SO_2Ph$ |
| 4-9 | CH=CH | $SO_2$(4-Me-Ph) |
| 4-10 | CH=CH | $SO_2$(4-Cl-Ph) |
| 4-11 | CH=C(Me) | CHO |
| 4-12 | CH=C(Me) | CN |
| 4-13 | CH=C(Me) | $CO_2Me$ |
| 4-14 | CH=C(Me) | $CO_2Et$ |
| 4-15 | CH=C(Me) | $SO_2Ph$ |
| 4-16 | CH=C(Me) | $SO_2$(4-Me-Ph) |
| 4-17 | CH=C(CN) | CN |
| 4-18 | CH=C(CN) | $CO_2Me$ |
| 4-19 | CH=C(CN) | $CO_2Et$ |
| 4-20 | CH=C(CN) | $SO_2Ph$ |
| 4-21 | CH=C(CN) | $SO_2$(4-Me-Ph) |
| 4-22 | CH=C(SMe) | $SO_2$(4-Me-Ph) |
| 4-23 | CH=C($CO_2Me$) | $CO_2Me$ |
| 4-24 | CH=C($CO_2Et$) | $CO_2Et$ |
| 4-25 | CH=C(Ph) | CN |
| 4-26 | CH=C(Ph) | $CO_2Me$ |
| 4-27 | CH=C(Ph) | $CO_2Et$ |
| 4-28 | $CH_2CH_2$ | OH |
| 4-29 | $CH_2CH_2$ | $NH_2$ |
| 4-30 | C(=$CH_2$) | CHO |
| 4-31 | C(=$CH_2$) | CN |
| 4-32 | C(=$CH_2$) | $CO_2Me$ |
| 4-33 | C(=$CH_2$) | $CO_2Et$ |
| 4-34 | CH=N | H |
| 4-35 | CH=N | Me |
| 4-36 | CH=N | Et |
| 4-37 | CH=N | n-Pr |
| 4-38 | CH=N | n-Bu |
| 4-39 | CH=N | Ph |

Table 4 continued

| Compound No. | Q b | M b |
|---|---|---|
| 4-40 | CH=N | OMe |
| 4-41 | CH=N | OEt |
| 4-42 | CH=N | $OCH_2CH=CH_2$ |
| 4-43 | CH=N | $OCH_2Ph$ |
| 4-44 | CH=N | NHMe |
| 4-45 | CH=N | NHEt |
| 4-46 | CH=N | NH(n-Pr) |
| 4-47 | CH=N | $N(Me)_2$ |
| 4-48 | CH=N | NHPh |
| 4-49 | CH=N | N(Me)Ph |
| 4-50 | CH=N | N=CHMe |
| 4-51 | CH=N | N=CHEt |
| 4-52 | CH=N | $N=C(Me)_2$ |
| 4-53 | CH=N | N=C(Me)Ph |
| 4-54 | CH=N | OH |
| 4-55 | CH=N | $NH_2$ |
| 4-56 | C(Me)=N | H |
| 4-57 | C(Me)=N | Me |
| 4-58 | C(Me)=N | Et |
| 4-59 | C(Me)=N | Ph |
| 4-60 | C(Me)=N | OH |
| 4-61 | C(Me)=N | OMe |
| 4-62 | C(Me)=N | OEt |
| 4-63 | C(Me)=N | O(n-Pr) |
| 4-64 | C(Me)=N | $OCH_2CH=CH_2$ |
| 4-65 | C(Me)=N | $OCH_2Ph$ |
| 4-66 | C(Me)=N | $NH_2$ |
| 4-67 | C(Me)=N | NHMe |
| 4-68 | C(Me)=N | $N(Me)_2$ |
| 4-69 | C(Me)=N | NHPh |
| 4-70 | C(Me)=N | N(Me)Ph |
| 4-71 | C(Me)=N | N=CHMe |
| 4-72 | C(Me)=N | N=CHPh |
| 4-73 | C(Me)=N | $N=C(Me)_2$ |
| 4-74 | C(Me)=N | N=C(Me)Ph |
| 4-75 | C(Ph)=N | H |
| 4-76 | C(Ph)=N | Me |
| 4-77 | C(Ph)=N | Et |

Table 4 continued

| Compound No. | Qb | Mb |
|---|---|---|
| 4-78 | C(Ph)=N | Ph |
| 4-79 | C(Ph)=N | OH |
| 4-80 | C(Ph)=N | OMe |
| 4-81 | C(Ph)=N | OEt |
| 4-82 | C(Ph)=N | $OCH_2CH=CH_2$ |
| 4-83 | C(Ph)=N | $OCH_2Ph$ |
| 4-84 | C(Ph)=N | $NH_2$ |
| 4-85 | C(Ph)=N | NHMe |
| 4-86 | C(Ph)=N | NHEt |
| 4-87 | C(Ph)=N | $N(Me)_2$ |
| 4-88 | C(Ph)=N | NHPh |
| 4-89 | C(Ph)=N | N(Me)Ph |
| 4-90 | C(Ph)=N | N=CHMe |
| 4-91 | C(Ph)=N | N=CHEt |
| 4-92 | C(Ph)=N | $N=C(Me)_2$ |
| 4-93 | C(Ph)=N | N=CHPh |
| 4-94 | C(Ph)=N | N=C(Me)Ph |
| 4-95 | - | U1 |
| 4-96 | - | U2 |
| 4-97 | - | U3 |
| 4-98 | - | U4 |
| 4-99 | - | U5 |
| 4-100 | - | U6 |
| 4-101 | - | U7 |
| 4-102 | - | U8 |
| 4-103 | - | U9 |
| 4-104 | - | U10 |
| 4-105 | - | U11 |
| 4-106 | - | U12 |
| 4-107 | - | U13 |
| 4-108 | - | U14 |
| 4-109 | - | U15 |
| 4-110 | - | U16 |
| 4-111 | - | U17 |
| 4-112 | - | U18 |
| 4-113 | - | U19 |
| 4-114 | - | U20 |
| 4-115 | - | U21 |

Table 4 continued

| Compound No. | Q b | M b |
|---|---|---|
| 4-116 | - | U22 |
| 4-117 | - | U23 |
| 4-118 | - | U24 |
| 4-119 | - | U25 |
| 4-120 | - | U26 |
| 4-121 | - | U27 |
| 4-122 | $CH_2$ | U1 |
| 4-123 | $CH_2$ | U2 |
| 4-124 | $CH_2$ | U3 |
| 4-125 | $CH_2$ | U4 |
| 4-126 | $CH_2$ | U5 |
| 4-127 | $CH_2$ | U6 |
| 4-128 | $CH_2$ | U7 |
| 4-129 | $CH_2$ | U8 |
| 4-130 | $CH_2$ | U9 |
| 4-131 | $CH_2$ | U10 |
| 4-132 | $CH_2$ | U11 |
| 4-133 | $CH_2$ | U12 |
| 4-134 | $CH_2$ | U13 |
| 4-135 | $CH_2$ | U14 |
| 4-136 | $CH_2$ | U15 |
| 4-137 | $CH_2$ | U16 |
| 4-138 | $CH_2$ | U17 |
| 4-139 | $CH_2$ | U18 |
| 4-140 | $CH_2$ | U19 |
| 4-141 | $CH_2$ | U20 |
| 4-142 | $CH_2$ | U21 |
| 4-143 | $CH_2$ | U22 |
| 4-144 | $CH_2$ | U23 |
| 4-145 | $CH_2$ | U24 |
| 4-146 | $CH_2$ | U25 |
| 4-147 | $CH_2$ | U26 |
| 4-148 | $CH_2$ | U27 |
| 4-149 | CH=N | $OCH_2CO_2H$ |
| 4-150 | CH=N | $OCH_2CO_2Me$ |
| 4-151 | CH=N | $OCH_2CO_2Et$ |
| 4-152 | C(Me)=N | $OCH_2CO_2Me$ |
| 4-153 | C(Me)=N | $OCH_2CO_2Et$ |

Table 4 continued

| Compound No. | Q b | M b |
|---|---|---|
| 4-154 | CH=C(SOMe) | $SO_2$(4-Me-Ph) |
| 4-155 | CH=($SO_2$Me) | $SO_2$(4-Me-Ph) |

In Table 4, U1 to U27 denote following structures.

Ul ; 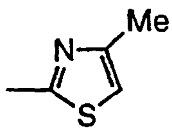

U2 ; 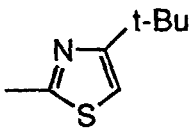

U3 ; 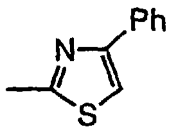

U4 ; 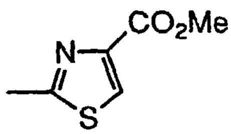

U5 ; 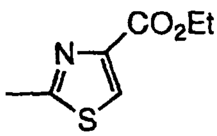

U6 ; 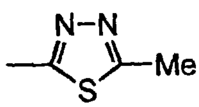

U7 ; 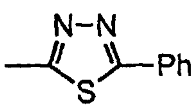

U8 ; 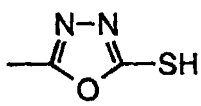

U9 ; 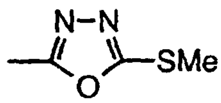

U10 ; 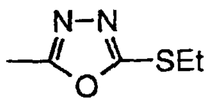

U11 ; 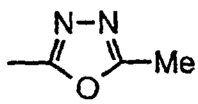

U12 ; 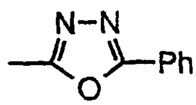

U13 ; 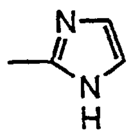

U14 ; 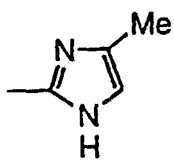

U15 ; 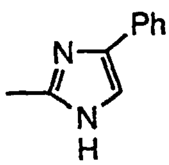

U16 ; 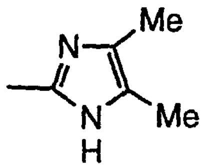

U23 ; 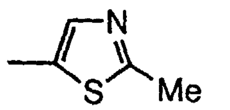

U17 ; 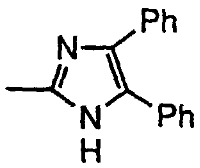

U24 ; 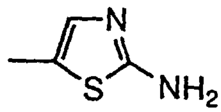

U18 ; 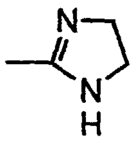

U25 ; 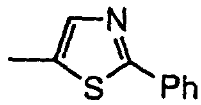

U19 ; 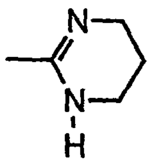

U26 ; 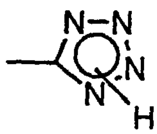

U20 ; 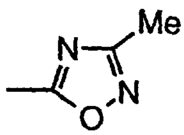

U27 ; 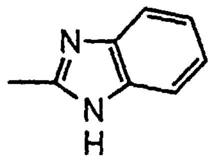

U21 ; 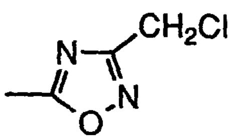

U22 ; 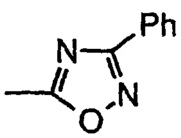

Next, the preparation methods of the compound of the general formula (1) according to the invention will be explained as follows.

(Method 1)

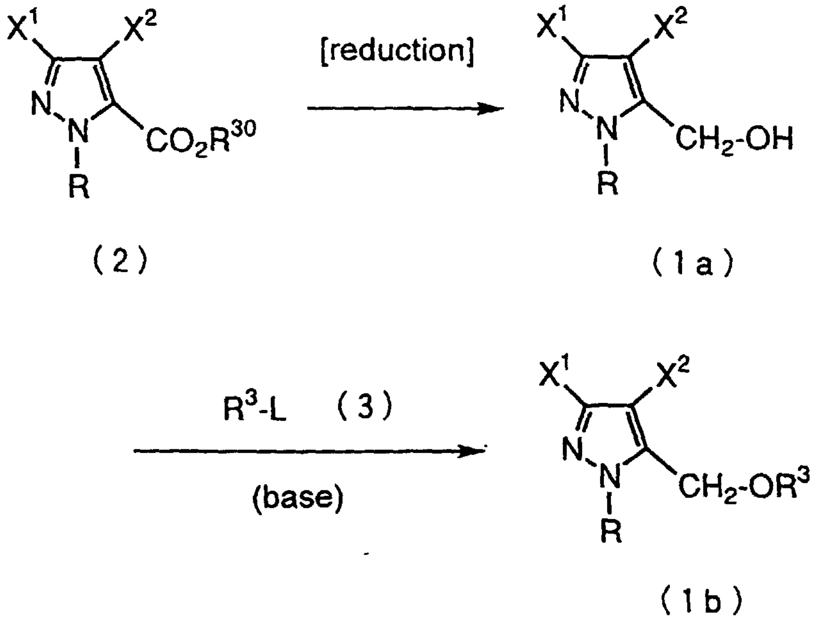


(2) (1a) (1b)

wherein, $X^1$, $X^2$, R and $R^3$ have same meanings as above, $R^{30}$ is H or $C_1$-$C_6$ alkyl, L is halogen, $OSO_2Me$, $OSO_2$(4-Me-Ph), $OSO_2CF_3$, $OCOR^{31}$ or the like, and $R^{31}$ is $C_1$-$C_{12}$ alkyl, $C_1$-$C_{10}$ haloalkyl, aryl or benzyl.

(Method 2)

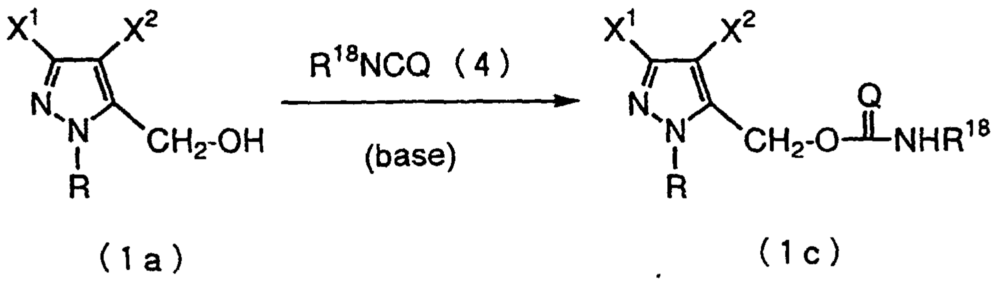


(1a) (1c)

wherein, $X^1$, $X^2$, R, $R^{18}$and Q have same meanings as above.

(Method 3)

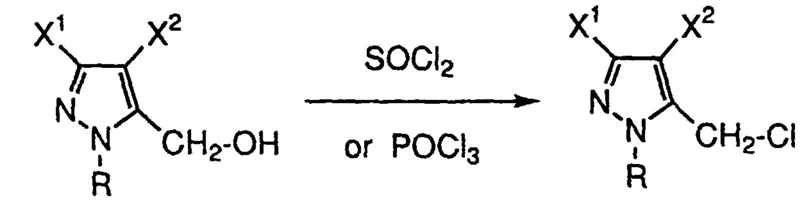


(1a) (5)

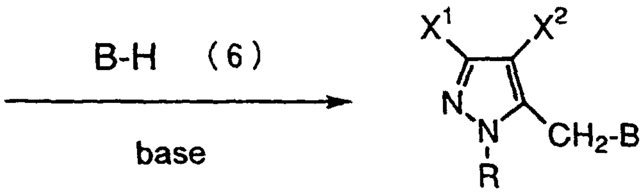


(1d)

wherein, $X^1$, $X^2$, R and B have same meanings as above.

(Method 4)

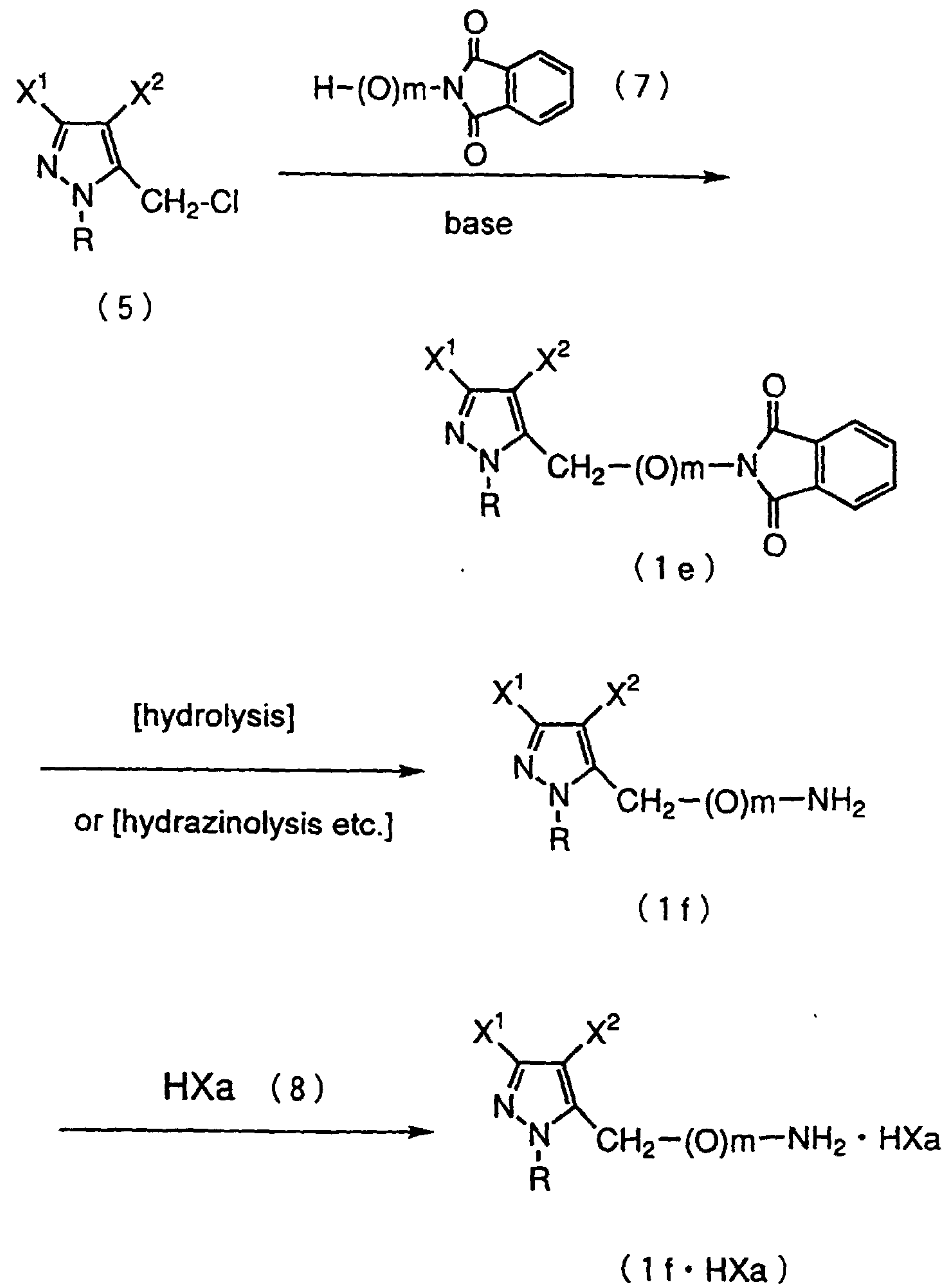


(5) (7) (1e) (1f) (1f · HXa)

wherein, $X^1$, $X^2$, R and m have same meanings as above, and Xa is halogen.

(Method 5)

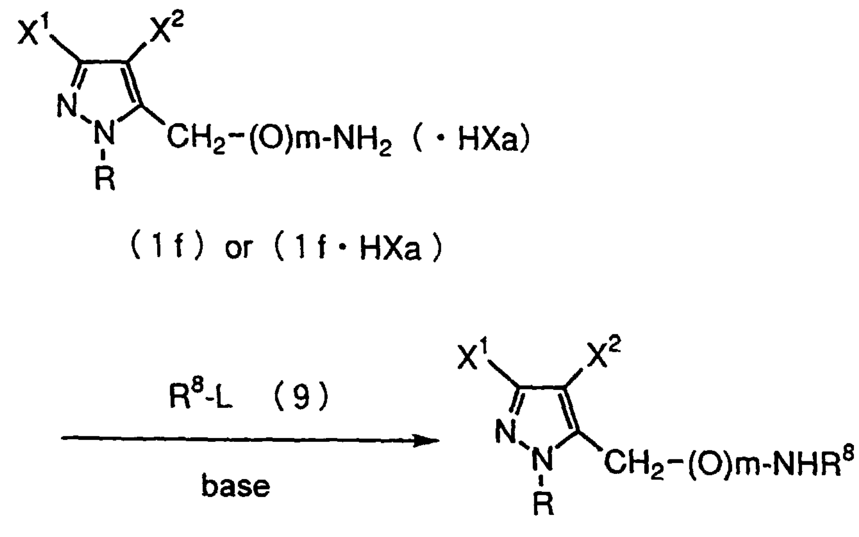


(1f) or (1f・HXa)

(1g)

wherein, $X^1$ $X^2$, R, m and $R^8$ have same meanings as above, and L is eliminating group such as halogen and the like and Xa is halogen.

(Method 6)

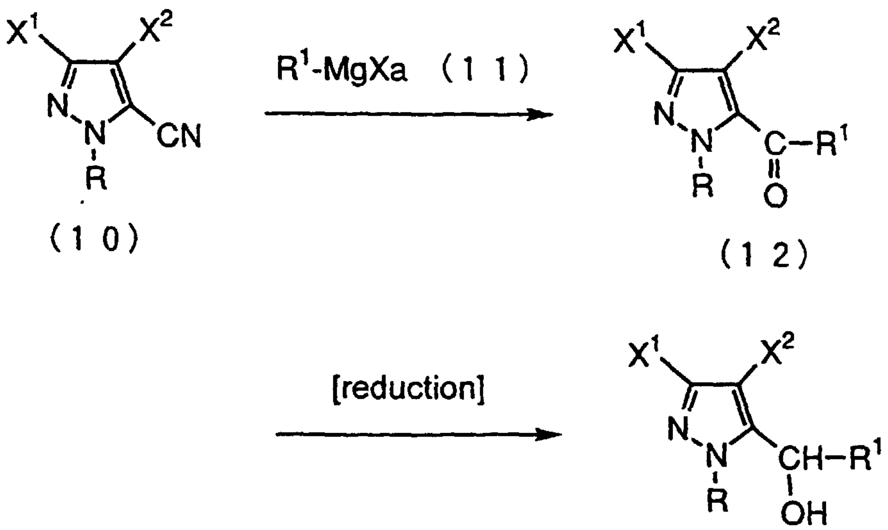


(10)

(12)

(1h)

wherein, $X^1$, $X^2$, R and $R^1$ have same meanings as above, and Xa is halogen.

(Method 7)

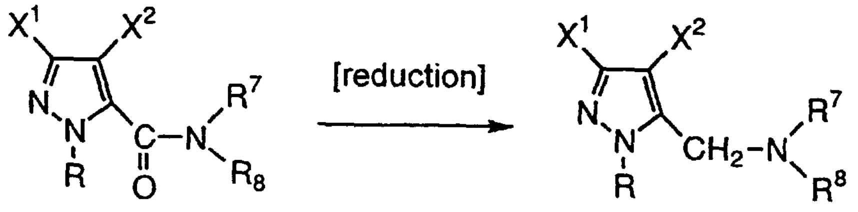


(1 3) (1 i)

wherein, $X^1$, $X^2$, R, $R^7$ and $R^8$ have same meanings as above.

(Method 8)

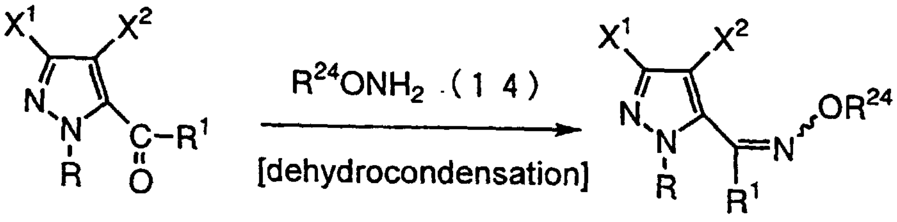


(1 2) (1 j)

wherein, $X^1$, $X^2$, R, $R^1$ and $R^{24}$ have same meanings as above.

(Method 9)

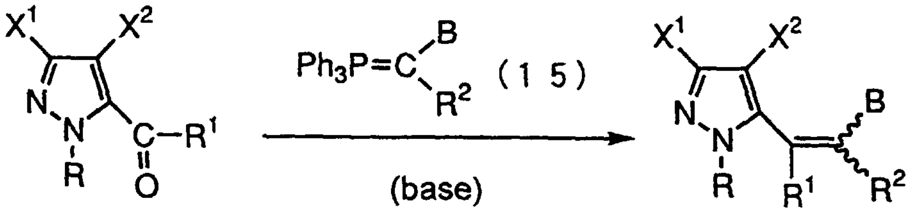


(1 2) (1 k)

wherein, $X^1$ $X^2$, R, $R^1$, $R^2$ and B have same meanings as above.

(Method 10)

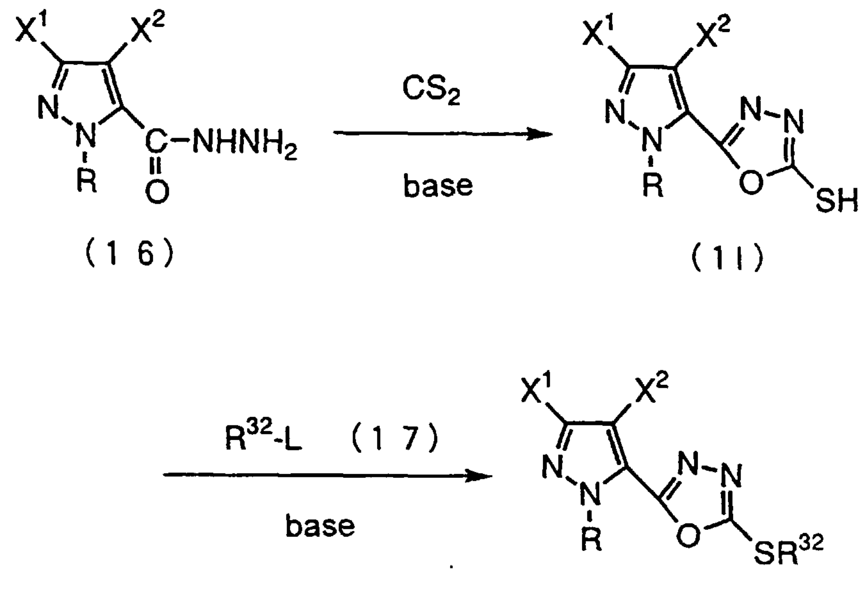


(1 6) (1 1)

(1 m)

wherein, $X^1$, $X^2$and R have same meanings as above, L is eliminating group such as halogen and the like, and $R^{32}$ is $C_1$-$C_4$ alkyl.

In Method 1, an alcohol compound (1a) according to the invention can be obtained by treating a pyrazole carboxylic acid ester (2) with a reducing agent in a solvent. As the solvent, there may be used, for example, mixed solvents of ethers such as diethyl ether, tetrahydrofuran and dimethoxyethane; aromatic hydrocarbons such as toluene and xylene; halogenated hydrocarbons such as dichloromethane, chloroform and 1,2-dichloroethane; alcohols such as methanol and ethanol. As the reducing agent, there may be used sodium boron hydride and lithium aluminum hydride, which are used within a range of 1 - 5 equivalents based on the ester (2). The reaction temperature may be in the range from 0 °C to the boiling point of the solvent.

Subsequently, the compound (1b) according to the invention can be obtained by reacting the alcohol compound (1a) with the $R^3$-L (3) in a solvent, optionally under the presence of a base. As the solvent, there may be anyone which is inert to the reaction, for example, ethers such as tetrahydrofuran, dioxane and dimethoxyethane; halogenated hydrocarbons such as dichloromethane, chloroform and 1,2-dichloroethane; nitriles such as acetonitrile and propionitrile; ketones such as acetone, methylethylketone and methylisobutylketone; aromatic hydrocarbons such as toluene and xylene; and amides such as N,N-dimethylformamide and N-methylpyrrolidone. As the base, there may be used inorganic bases (such as potassium carbonate, sodium carbonate, sodium hydrogencarbonate, sodium hydroxide, potassium hydroxide and sodium hydride), organic bases (such as pyridine, triethylamine and tributylamine) and alkoxides (such as potassium t-butoxide), which are used within a range of 0.01 - 5 equivalents. The reaction temperature may be in the range from 0 °C to the boiling point of the solvent.

In Method 2, the carbamate or thiocarbamate compound (1c) according to the invention can be obtained by reacting the alcohol compound (1a) with the isocyanate or thioisocyanate compound (4) in a solvent. As the solvent, there may be anyone which is inert to the reaction, for example, ethers such as tetrahydrofuran, dioxane and dimethoxyethane; halogenated hydrocarbons such as dichloromethane, chloroform and 1,2-dichloroethane; nitriles such as acetonitrile and propionitrile; ketones such as acetone, methylethylketone and methylisobutylketone; and aromatic hydrocarbons such as toluene and xylene. Optionally, a base (such as triethylamine and pyridine) may be added in an amount of 0.01 -3 equivalents to carry out the reaction. The reaction temperature may be in the range from —20 °C to the boiling point of the solvent.

In Method 3, the chloro compound (5) can be obtained by reacting the alcohol compound (1a) with a thionyl chloride or phosphorous oxychloride. For this case, the reaction may be carried out with a solvent. As the solvent, there may be anyone which is inert to the reaction, for example, halogenated hydrocarbons such as dichloromethane, chloroform and 1,2-dichloroethane; nitrites such as acetonitrile and propionitrile; ketones such as acetone, methylethylketone and methylisobutylketone; and aromatic hydrocarbons such as toluene and xylene. Thionyl chloride or

phosphorous oxychloride may be used in the range of 1 - 10 equivalents based on (1a). The reaction temperature may be in the range from 0 °C to the boiling point of the solvent.

Subsequently, the compound (1d) according to the invention can be obtained by reacting the chloro compound (5) with the B-H (6) in a solvent under the presence of a base. As the base, there may be used inorganic bases (such as potassium carbonate, sodium carbonate, sodium hydrogencarbonate, sodium hydroxide, potassium hydroxide and sodium hydride), organic bases (such as pyridine, triethylamine and tributylamine) and alkoxides (such as potassium t-butoxide and sodium methoxide), which are used in the range of 0.01 - 5 equivalents. As the solvent, there may be anyone which is inert to the reaction, for example, ethers such as tetrahydrofuran, dioxane and dimethoxyethane; halogenated hydrocarbons such as dichloromethane, chloroform and 1,2-dichloroethane; nitriles such as acetonitrile and propionitrile; aromatic hydrocarbons such as toluene and xylene; and amides such as N,N-dimethylformamide and N-methylpyrrolidone. The reaction temperature may be in the range from 0 °C to the boiling point of the solvent.

In Method 4, the compound (1e) according to the invention can be obtained by reacting the chloro compound (5) with the phthalimide or N-hydroxy phthalimide (7) in a solvent under the presence of a base. As the base, there may be used potassium carbonate, sodium carbonate, sodium hydroxide, potassium hydroxide, sodium hydride and the like, which are used in the range of 1 - 5 equivalents. As the solvent, there may be anyone which is inert to the reaction, for example, ethers such as tetrahydrofuran, dioxane and dimethoxyethane; and amides such as N,N-dimethylformamide and N-methylpyrrolidone. The reaction temperature may be in the range from a room temperature to the boiling point of the solvent.

Subsequently, the compound (1f) according to the invention can be obtained by hydrolysis or hydrazinolysis in Gabriel synthesis, namely by subjecting to a hydrohalogenic acid (such as hydrochloric acid), alkali hydroxide (such as sodium hydroxide and potassium hydroxide), hydrazine hydrate or hydroxylamine in a suitable solvent. As the solvent, there may be used for example water; alcohols such as methanol and ethanol; amides such as N,N-dimethylformamide; and mixtures thereof. The reaction temperature may be in the range from the room temperature to the boiling point of the solvent.

Further, the compound (1f • HXa): a mineral salt corresponding to (1f), according to the invention can be obtained by reacting the compound (1f) according to the invention with the mineral acid (8) in a solvent. As the solvent, there may be anyone which is inert to the reaction, for example, ethers such as tetrahydrofuran, dioxane and dimethoxyethane; halogenated hydrocarbons such as dichloromethane, chloroform and 1,2-dichloroethane; ketones such as acetone; aromatic hydrocarbons such as toluene; alcohols such as methanol and ethanol; water; and mixtures thereof. The reaction temperature may be in the range from 0 °C to the boiling point of the solvent.

In Method 5, the compound (1g) according to the invention can be obtained by reacting the compound (1f) or its salt (1f • HXa) according to the invention with the $R^8$-L (9) in a solvent under the presence of a base. As the base, there may be used inorganic bases (such as potassium carbonate, sodium carbonate, sodium hydrogencarbonate, sodium hydroxide, potassium hydroxide and sodium hydride), organic bases (such as pyridine, triethylamine and tributylamine) and alkoxides (such as potassium t-butoxide), which are used in the range of 0.01 - 5 equivalents. As the solvent, there may be anyone which is inert to the reaction, for example, ethers such as tetrahydrofuran, dioxane and dimethoxyethane; halogenated hydrocarbons such as dichloromethane, chloroform and 1 2-dichloroethane; nitriles such as acetonitrile and propionitrile; ketones such as acetone, methylethylketone, methylisobutylketone; aromatic hydrocarbons such as toluene and xylene; and amides such as N,N-dimethylformamide and N-methylpyrrolidone. The reaction temperature may be in the range from 0 °C to the boiling point of the solvent.

In Method 6, the carbonyl compound (12) can be obtained by reading the nitrile compound (10) with the Grignard reagent (11) in a solvent.

As the solvent, there may be used, for example, ethers such as diethylether, tetrahydrofuran, dioxane and dimethoxyethane. The reaction temperature may be in the range from 0 °C to the boiling point of the solvent.

Subsequently, the compound (1h) according to the invention can be obtained by treating the carbonyl compound (12) in a solvent with a reducing agent in reduction reaction. As the solvent, there may be used, for example, ethers such as diethylether, tetrahydrofuran and dimethoxyethane; aromatic hydrocarbons such as toluene and xylene; halogenated hydrocarbons such as dichloromethane, chloroform and 1,2-dichloroethane; alcohols such as methanol and ethanol; and mixtures thereof. As the reducing agent, there may be used metal hydrides such as sodium boron hydride and lithium aluminum hydride; and boron compounds such as diborane, borane-pyridine complex and borane-t-butylamine complex, which is used in the range or 0.25-2 equivalents. The reaction temperature may be in the range from —50 °C to the boiling point of the solvent.

In Method 7, the compound (1i) according to the invention can be obtained by reacting the pyrazole carboxylic acid amide compound (13) with a reducing agent in a solvent. As the reducing agent, there is no limitation as long as which can reduce amide into amine, for example, metal hydrides such as lithium aluminum hydride, sodium boron hydride and borane may be used. Alternatively, reduction can be carried out by treating with metallic sodium in an alcohol as the solvent. The reducing agent may be used in an amount of 1 equivalent or more. Further, as the suitable solvent in the reaction, there may be mentioned ethers such as diethyl ether, tetrahydrofuran and dimethoxyethane;

aromatic hydrocarbons such as toluene and xylene, and as the alcohol such as isoamyl alcohol. The reaction temperature may be in the range from the room temperature to the boiling point of the solvent.

In Method 8, the compound (1j) according to the invention can be obtained by reacting the carbonyl compound (12) with the hydroxylamine compound (14) in a solvent to dehydrate and condensate them. As the solvent, there may be mentioned alcohols such as methanol, ethanol and n-butanol; water; or mixtures thereof. The reaction temperature is suitably the reflux temperature of the solvent.

In Method 9, the compound (1k) according to the invention can be obtained by reacting the carbonyl compound (12) with the phosphorane compound (15) in a solvent, optionally under the presence of a base to condense them. As the base, there may be used inorganic bases (such as potassium carbonate, sodium carbonate, sodium hydrogencarbonate, sodium hydroxide, potassium hydroxide and sodium hydride), organic bases (such as pyridine, triethylamine and tributylamine) and alkoxides (such as potassium t-butoxide), which are used in the range of 0.01 - 5 equivalents. As the solvent, there may be anyone which is inert to the reaction, for example, ethers such as tetrahydrofuran, dioxane and dimethoxyethane; aromatic hydrocarbons such as toluene and xylene; and amides such as N,N-dimethylformamide and N-methylpyrrolidone. The reaction temperature may be in the range from 0 °C to the boiling point of the solvent.

In Method 10, the compound (1l) according to the invention can be obtained by cyclizing the carboxylic hydrazide compound (16) with carbon disulfide in a solvent under the presence of a base. As the base, there may be used inorganic bases (such as potassium carbonate, sodium carbonate, sodium hydrogencarbonate, sodium hydroxide, potassium hydroxide and sodium hydride), organic bases (such as pyridine, triethylamine and tributylamine) and alkoxides (such as potassium t-butoxide), which are used in the range of 0.01 - 5 equivalents. As the solvent, there may be anyone which is inert to the reaction, for example, ethers such as tetrahydrofuran, dioxane and dimethoxyethane; aromatic hydrocarbons such as toluene and xylene; alcohols such as methanol and ethanol; water; or mixtures thereof The reaction temperature may be in the range from 0 °C to the boiling point of the solvent.

Subsequently, the compound (1m) according to the invention can be obtained by reacting thus obtained compound (1l) according to the invention with the $R^{32}$-L (17) in a solvent under the presence of a base. As the base, there may be used inorganic bases (such as potassium carbonate, sodium carbonate, sodium hydrogencarbonate, sodium hydroxide, potassium hydroxide and sodium hydride), organic bases (such as pyridine, triethylamine and tributylamine) and alkoxides (such as potassium t-butoxide), which are used in the range of 0.01 - 5 equivalents. As the solvent, there may be anyone which is inert to the reaction, for example, ethers such as tetrahydrofuran, dioxane and dimethoxyethane; aromatic hydrocarbons such as toluene and xylene; and amides such as N,N-dimethylformamide and N-methylpyrrolidone. The reaction temperature may be in the range from 0 °C to the boiling point of the solvent.

For reference, the pyrazole carboxylate of the general formula (2) is known compound from Japanese Patent Application Laid-Open Hei 8-12510. Further, the pyrazole carboxylic acid amide compound (13) and the hydrazide compound (16) are known compounds from Japanese Patent Application Laid-Open Hei 9-176125. Still further, the nitrile compound of the general formula (10) can be prepared by subjecting a pyrazole carboxylic acid amide compound to known dehydration reaction. A pyrazole compound halogenated at 4-position can be synthesized by reacting corresponding pyrazole carboxylic acid or pyrazole carboxylic acid amide compound with general halogenating agents (such as sulfuryl chloride, N-chlorosuccinimide, N-bromosuccinimide, bromine, iodine, t-butyl hypochlorite) and then carrying out the above-mentioned preparation methods.

As for the plant diseases which are controlled by the compound according to the invention, there may be mentioned as follows:

rice blast (Pyricularia oryzae), helminthosporium leaf spot (Cochliobolms miyabeanus), sheath blight (Rhizoctonia solani),
barley and wheat; powdery mildew (Erysiphe graminis f. sp. hordei, f. sp. tritici), stripe (Pyrenophora graminea), net blotch (Pyrenophora teres), scab (Gibberella zeae), rust (Puccinia striiformis, R graminis, P. recondita, P. hordei), snow blight (Tipula sp., Micronectria nivalis), loose smut (Ustilago tritici, U. nuda), eye spot (Pseudocercosporella herpotrichoides), scald (Rhynchosporium secalis), spekled leaf blotch (Septoria tritici), glume blotch (Leptosphaeria nodorum),
Citrus; melanose (Diaporthe citri), scab (Elsinoe fawcetti), green mold and blue mold (Penicillium digitalum, P. italicum),
Apple; blossom blight (Sclerotinia mali), canker (Valsa mali), powdery mildew (Podosphaera lcuchotricha), alternaria leaf spot (Alternaria mali), scab (Venturia inaequalis),
Pear; scab (Venturia nashicola), black spot (Alternaria kikuchiana), rust (Gymnosporangium haraenum),
Peach; brown rot (Sclerotinia cinerea), scab (Cladosporium carpophilum), phomopsis rot (Phomopsis sp.),
Grape; downy mildew (Plasmopara viticola), anthracnose (Elsinoe ampelina), ripe rot (Glomerella cingulate), powdery mildew (Uncinula necator), rust (Phakopsora ampelopsidis),
Kaki; anthracnose (Gloeosporium kaki), angular leaf spot and circular leaf spot (Cercospora kaki, Mycosphaerella

hawae),
Melons; downy mildew (Pseudoperonospora cubensis), anthracnose (Colletotrichum lagenarium), powdery mildew (Sphaerotheca fuliginea), gummy stem blight (Mycosphaerella melonis),
Tomato; late blight (Phytophthora infestans), early blight (Alternaria solani), leaf mold (Cladosporium fulvum),
Egg plant; brown spot (Phomopsis vexans), powdery mildew (Erysiphe cichoracoarum),
Rape; black rot (Alternaria japonica), white rot (Cercosporella brassicae),
Onion; rust (Puccinia allii),
Soybean; purple stain of seed (Cercospora kikuchii), sphaceloma scab (Elisinoe glycines), black spot (Diaporthe phaseololum),
Kidney bean; anthracnose (Colletotrichum lindemuthianum),
Peanut; leaf spot (Mycosphaerella personatum), brown leaf spot (Cercospora arachidicola),
Pea; powdery mildew (Erysiphe pisi),
Potato; late blight (Alternaria solani),
Strawberry; powdery mildew (Sphaerotheca humuli),
Tea plant; net blister blast (Exobasidium reticulatum), white scab (Elsinoe leucospila),
Tobacco; brown spot (Alternaria lingipes), powdery mildew (Erysiphe cichoracearum), anthracnose (Colletotrichum tabacum),
Beet; cercospora leaf (Cercospora beticola),
Rose; black spot (Diplocarpon rosae), powdery mildew (Sphaerotheca pannosa),
Chrysanthemum; leaf spot (Septoria chrysanthemiindici), rust (Puccinia horiana),
Various crops; gray mold (Botrytis cinerea),
Various crops; sclerotinia rot (Sclerotinia sclerotiorum), and the like.

For use of the compound according to the invention as a plant disease control agent (a fungicide for agriculture and horticulture), the compound is mixed with a suitable solid carrier or liquid carrier, if desired with addition of a surfactant, a penetrant, a spreader, a thickner, an anti-freezer, a binder, an anti-solidifier, a disintegrating agent, an anti-decomposition agent or the like to make an optional pharmaceutical preparation type such as solution, emulsion, wettable powder, soluble powder, granular wettable powder, granular soluble powder, suspension, emulsion-suspension, suspoemulsion, microemulsion, dust, granule and gel for practical uses. Further, the preparations in above-mentioned types may be enclosed in a water-soluble envelop from the viewpoints of improvement in laborsaving and safety.

As solid carriers, there may be mentioned for example natural minerals such as quartz, kaolinite, pyrophyllite, sericite, talc, bentonite, acid clay; attapulgite, zeolite and diatomaceous earth; inorganic salts such as calcium carbonate, ammonium sulfate, sodium sulfate and potassium chloride, synthetic silicic acid and synthetic silicates.

As liquid carriers, there may be mentioned, for example, alcohols such as ethylene glycol, propylene glycol and isopropanol; aromatic hydrocarbons such as xylene, alkylbenzene and alkylnaphthalene; ethers such as butyl cellosolve; ketones such as cyclohexanone; esters such as γ-butylolactone; acid amides such as N-methylpyrrolidone and N-octylpyrrolidone; plant oils such as soybean oil, rape oil, cottonseed oil and castor oil; and water.

These solid and liquid carriers may be used alone or two or more carriers may be used in combination.

As the surfactant, there may be mentioned, for example, nonionic surfactants such as polyoxyethylene alkylether, polyoxyethylene alkylarylether, polyoxyethylene styrylphenylether, polyoxyethelenepolyoxypropylene block copolymer, polyoxyethylene aliphatic acid ester, sorbitan aliphatic acid ester and polyoxyethylene sorbitan aliphatic acid ester; anionic surfactants such as alkyl sulfate, alkyl benzenesulfonate, lignin sulfonate, alkyl sulfosuccinate, naphtalene sulfonate, alkylnaphtalene sulfonate, salt of formarin condensate of naphthalene sulfonate, salt of formarin condensate of alkylnaphthalene sulfonate, polyoxyethylene alkylarylether sulfate and phosphate, polyoxyethylene styrylphenylether sulfate and phosphate, polycarboxylate and polystyrene sulfonate; cationic surfactants such as alkyl amine salt and alkyl quartery ammonium salt, as well as amphoteric surfactants such as amino acid type and betaine type.

A content of the surfactant is not limited specifically, but it is desirably 0.05 - 20 parts by weight per 100 parts by weight of the formulation according to the invention. Further, the surfactants may be used alone or two or more surfactants may be used in combination.

Formulation examples of the preparations using the compounds according to the invention are shown as follows. However, it is not limited thereto. In the following blending examples, "part" means "parts by weight".

[Wettable powder]

| | |
|---|---|
| compound according to the invention | 0.1 - 80 parts |
| solid carrier | 5 - 98.9 parts |
| surfactant | 1 - 10 parts |
| others | 0 - 5 parts |

There may be mentioned as others for example an anti-solidifier and an anti-decomposition agent and the like.

[Emulsion]

| | |
|---|---|
| compound according to the invention | 0.1 - 30 parts |
| liquid carrier | 45 - 95 parts |
| surfactant | 4.9 - 15 parts |
| others | 0 - 10 parts |

There may be mentioned as others for example a spreader and an anti-decomposition agent and the like.

[Suspension]

| | |
|---|---|
| compound according to the invention | 0.1 - 70 parts |
| liquid carrier | 15 - 98.89 parts |
| surfactant | 1 - 12 parts |
| others | 0.01 - 30 parts |

There may be mentioned as others for example an anti-freezer and a thickner and the like.

[Granular wettable powder]

| | |
|---|---|
| compound according to the invention | 0.1 - 90 parts |
| solid carrier | 0 - 98.9 parts |
| surfactant | 1 - 20 parts |
| others | 0 - 10 parts |

There may be mentioned as others for example a binder and an anti-decomposition agent and the like.

[Solution]

| | |
|---|---|
| compound according to the invention | 0.01 - 70 parts |
| liquid carrier | 20 - 99.99 parts |
| others | 0 - 10 parts |

There may be mentioned as others for example an anti-freezer and a spreader and the like.

[Granulate]

| | |
|---|---|
| compound according to the invention | 0.01 - 80 parts |
| solid carrier | 10 - 99.99 parts |
| others | 0 - 10 parts |

There may be mentioned as others for example a binder and an anti-decomposition agent and the like.

[Dust]

| | |
|---|---|
| compound according to the invention | 0.01 - 30 parts |
| solid carrier | 65 - 99.99 parts |
| others | 0 - 5 parts |

There may be mentioned as others for example an anti-drifting agent and an anti-decomposition agent and the like.

In use, the above-mentioned agent may be sprayed after diluting with water to 1 - 10000 times or without dilution.

As application method of the compound according to the invention, there may be mentioned foliar application, soil treatment and seed disinfection.

Further, if necessary, other herbicides, various insecticides, fungicides, plant growth control agents, synergists may be mixed and used together at preparation or spraying. An application rate of the compound according to the invention varies due to an application field, an application period, an application method, a target disease, a cultured crop and the like, but generally it is suitable to apply about 0.005 - 50 kg calculated as the active ingredient per 1 hectare.

Examples

Synthesis examples of the compounds according to the invention are shown in the following examples, but the invention is not limited thereto.

[Example 1]

Synthesis of 3-chloro-1-methyl-5-hydroxymethylpyrazole (Compound No.1-1(a) according to the invention)

37.79(0.2 mol) of ethyl 3-chloro-1-methylpyrazole-5-carboxylate and 19.0 g (0.5 mol) of sodium boron hydride were suspended in 800 ml of tetrahydrofuran. 160 ml of methanol was added to the reaction mixture over 1 hour with reflux, and heated under reflux for further 1 hour with stirring. The reaction solution was cooled to the room temperature, to which 200 ml of water was added. The reaction solvent was distilled off under reduced pressure, and the residue was extracted with chloroform and dried over anhydrous sodium sulfate. After filtration, 27.92 g of the objective compound No.1-1 (a) according to the invention was obtained as white crystals by distilling off the solvent under reduced pressure.

Melting point: 95 - 96.5° C

[Example 2]

Synthesis of (3-chloro-1 -methylpyrazol-5-yl)methylaceteate (Compound No.1-55(a) according to the invention)

0.35 g (4.4 mmol) of acetyl chloride was added dropwise to a mixture of 0.59 g (4.0 mmol) of 3-chloro-1-methyl-5-hydroxymethylpyrazole, 0.49 g(4.8 mmol) of triethylamine and 4 ml of tetrahydrofuran with water cooling. After stirring at the room temperature for 12 hours, the solvent was distilled off under reduced pressure. 10 ml of water was added to the residue, and the residue was extracted with chloroform and dried over anhydrous sodium sulfate. After filtration, 0.6 g of the objective compound No.1-55(a) according to the invention was obtained as colorless oil by distilling off the solvent under reduced pressure and purifying the residue with a silica gel column chromatography (chloroform).

Refractive index $n_{D21\cdot9}$ 1.4934

[Example 3]

Synthesis of (3-chloro-1-methylpyrazol-5-yl)methyl-N-phenylcarbamate (Compound No.1-93(a) according to the invention)

1.31 g (0.11 mol) of phenyl isocyanate was added dropwise to a mixture of 1.46 g (0.10 mol) of 3-chloro-1-methyl-5-hydroxymethylpyrazole and 100 ml of tetrahydrofuran with ice cooling. After stirring at the room temperature for 10 minutes, the solvent was distilled off. 2.1 g of the objective compound No.1-93(a) according to the invention was obtained as white crystal by filtering off precipitated crystal and washing with diethylether and n-hexane.

Melting point 165 - 168 °C.

[Example 4]

Synthesis of 3-chloro-1-methyl-5-phenoxymethylpyrazole (Compound No.1-26(a) according to the invention)

67.8 g (0.57 mol) of thionyl chloride was added dropwise to a mixture of 27.92 g (0.19 mol) of 3-chloro-1-methyl-5-hydroxymethylpyrazole and 280ml of chloroform with ice cooling. After stirring at the room temperature for 1 hour, 31.6 g of 3-chloro-5-chloromethyl-1-methylpyrazole was obtained as light yellow oil by distilling off the solvent and excess of thionyl chloride under reduced pressure.

0.28 g (3.0 mmol) of phenol was added dropwise at the room temperature to a suspension of 72 mg (3.0 mmol) of sodium hydride in 30 ml of tetrahydrofuran, and stirred for 30 minutes. Thereto, 0.5 g (3.0 mmol) of 3-chloro-5-chloromethyl-1-methylpyrazole was added and stirred at the room temperature for 12 hours. The reaction mixture was poured into 10 ml of water, extracted with chloroform and dried over anhydrous sodium sulfate. After filtration, 0.5 g of the objective compound No.1-26(a) according to the invention was obtained as colorless oil by distilling off the solvent under reduced pressure and purifying the residue with a silica gel column chromatography (chloroform).

Refractive index $n_{D21.9}$ 1.5284

[Example 5]

Synthesis of N-(3-chloro-1-methylpyrazol-5-yl)methylphthalimide (Compound No.3-2(a) according to the invention)

35.4 g (0.19 mol) of potassium phthalimide and 4.5 g (0.027 mol) of potassium iodide were added to a mixture of 31.6 g (0.19 mol) of 3-chloro-5-chloromethyl-1-methylpyrazole and 570 ml of N,N-dimethylformamide, and stirred at 80 °C for 1 hour. The reaction solution was cooled to the room temperature, poured into 1 L of ice water, and extracted with ethyl acetate. An organic layer was washed with 1 N aqueous sodium hydroxide solution followed by a saturated saline solution, and then dried over anhydrous magnesium sulfate. After filtration, 47.3 g of an objective compound No.3-2(a) according to the invention was obtained as white crystals by distilling off the solvent under reduced pressure.

Melting point: 147 - 148 °C.

[Example 6]

Synthesis of (3-chloro-1-methylpyrazol-5-yl)methylamine hydrochloride (Compound No.2-1(a) HCl according to the invention)

8.59 g (0.17 mol) of hydrazine monohydrate was added to a mixture of 7.3 g (0.17 mol) of N-(3-chloro-1-methylpyrazol-5-yl)methylphthalimide and 870 ml of ethanol, and heated under reflux for 1 hour with stirring. The reaction solution was cooled to the room temperature, then 17.9 g of concentrated hydrochloric acid and heated to reflux for 30 minutes with stirring. After allowing to cool, the solvent was distilled off under a reduced pressure, and 200 ml of water was added to the residue, and insolubles were filtered off. The water was distilled off from the filtrate under reduced pressure, and 27.49 g of Compound No.2-1 (a) HCl according to the invention was obtained as white crystals by filtering off precipitated crystal and washing with ethyl acetate.

Melting point 215 - 216 °C.

[Example 7]

Synthesis of N-((3-chloro-1 -methylpyrazol-5-yl)methyl)benzamide (Compound No.2-91(a) according to the invention)

0.56 g (4.0 mmol) of benzoyl chloride was added to a mixture of 0.73 g (4.0 mmol) of (3-chloro-1-methylpyrazol-5-yl)methylamine hydrochloride, 0.89 g (8.8 mmol) of triethylamine and 10 ml of tetrahydrofuran, and stirred at the room temperature for 12 hours. The solvent was distilled off under a reduced pressure, and 10 ml of water was added to the residue, which was extracted with chloroform and dried over anhydrous sodium sulfate. After filtration, 0.73 g of Compound No.2-91(a) according to the invention was obtained as white crystals by distilling off the solvent under reduced pressure and purifying the residue with a silica gel column chromatography (chloroform).

Melting point: 128 - 130 °C.

[Example 8]

Synthesis of N-((3-chloro-1-methylpyrazol-5-yl)methyl)trifluoroacetoamide (Compound No.2-87(a) according to the invention)

1.05 g (5.0 mmol) of trifluoroacetic anhydride was added dropwise to a mixture of 0.73 g (5.0 mmol) of (3-chloro-1-methylpyrazol-5-yl)methylamine and 10 ml of chloroform with ice cooling. After stirring at the room temperature for 1 hour, the solvent was distilled off under reduced pressure. 1.0 g of the compound No.2-87(a) according to the invention was obtained as white crystal by filtering off precipitated crystal and washing with n-hexane and diisopropyl ether.

Melting point: 90 - 92 °C.

[Example 9]

Synthesis of ((3-chloro-1-methylpyrazol-5-yl)methyl)phenylamine (Compound No.2-26(a) according to the invention)

1.0 g (6.1 mmol) of 3-chloro-5-chloromethyl-1-methylpyrazole was added dropwise to a mixture of 1.86 g (20 mmol) of aniline, 0.84 g (6.1 mmol) of anhydrous potassium carbonate and 8 ml of N,N-dimethylformamide, and stirred at the room temperature for 12 hours. The reaction solution was poured into 10 ml of water, extracted with ethyl acetate, washed with water and saturated saline solution, and thereafter over with anhydrous magnesium sulfate. After filtration, the solvent was distilled off under reduced pressure. 1.0 g of the compound No.2-26(a) according to the invention was obtained as white crystal by filtering off precipitated crystal and washing with n-hexane and diisopropyl ether.

Melting point: 123 - 124 °C.

[Example 10]

Synthesis of O-benzyl-3-chloro- 1-methylpyrazole-5-carboaldehyde oxime (Compound No.4-43(a) according to the invention)

0.73 g (5.0 mmol) of 3-chloro-1-methylpyrazole-5-carboaldehyde, 1.04 g (6.5 mmol) of O-benzyloxyamine hydrochloride and 0.53 g (6.6 mmol) of sodium acetate were dissolved in a mixed solvent of 20 ml of ethanol and 6 ml of water, and the mixture was heated under reflux for 1 hour with stirring. The reaction solution was cooled to the room temperature, and the solvent was distilled off under reduced pressure. 50 ml of water was added. Extraction with ethyl acetate was carried out, and an organic layer was washed with a saturated saline solution and thereafter dried over anhydrous magnesium sulfate. After filtration, 0.9 g of the compound No.4-43(a) according to the invention was obtained as colorless oil by distilling off the solvent under reduced pressure and purifying the residue with a silica gel column chromatography (n-hexane : ethyl acetate = 5 : 1).

Refractive index $n_{D24\cdot5}$ 1.5767

[Example 11]

Synthesis of 3-(3-chloro-1-methylpyrazol-5-yl)acrolein (Compound No.4-1(a) according to the invention)

2.55 g (7.61 mmol) of triphenylphosphoranylidene acetoaldehyde was added to a mixed solution of 1.0 g (6.92 mmol) of 3-chloro-1-methylpyrazole-5-carboaldehyde dissolved in 10 ml of tetrahydrofuran. Stirring was carried out at the room temperature for 18 hours. 10 ml of a saturated saline solution was added to the reaction solution, and then the reaction solution was extracted with ethyl acetate, washed with water, and thereafter dried over anhydrous magnesium sulfate. After filtration, 1.5 g of the compound No.4-1(a) according to the invention was obtained as white crystal by distilling off the solvent under reduced pressure and purifying the residue with a silica gel column chromatography (n-hexane: ethyl acetate = 10:1).

Melting point: 75 - 80 °C

[Example 12]

Synthesis of 3-chloro-1-methyl-5-(5-mercapto-1,3,4-oxadiazol-2-yl)pyrazole (Compound No.4-102(a) according to the invention)

15 ml of an aqueous solution in which 0.6 g of potassium hydroxide was dissolved was poured into a mixed solution of 2.51 g (14.4 mmol) of 3-chloro-1-methylpyrazole-5-carbohydrazide dissolved in 30 ml of ethanol, and then 1.2 ml of carbon disulfide was added. Stirring was carried out for 7 hours with heating to reflux. After cooling the reaction mixture, the solvent was distilled off under reduced pressure. 50 ml of water was added, and acetic acid was added to acidify pH of the mixed solution. 2.0g of the compound No.4-102(a) according to the invention was obtained as white crystal by filtering and drying precipitated crystal.

Melting point: 220 °C (decomposition)

[Example 13]

Synthesis of 3-chloro-1-methyl-5-(5-methylthio-1,3,4-oxadiazol-2-yl)pyrazole (Compound No.4-103(a) according to the invention)

2.07 g (15.0 mmol) of potassium carbonate and then 1.07 g (7.5 mmol) of methyl iodide were added to a mixed solution of 1.08 g (5.0 mmol) of 3-chloro-1-methyl-5-(5-mercapto-1,3,4-oxadiazol-2-yl)pyrazole dissolved in 30 ml of N,N-dimethylformamide. Heating was carried out at 50 °C for 1 hour with stirring. Under reduced pressure, the solvent in the reaction solution was distilled off. 20 ml of water was added, and then the reaction solution was extracted with ethyl acetate, washed with water, and thereafter dried over anhydrous magnesium sulfate. After filtration, 1.0 g of the compound No.4-103(a) according to the invention was obtained as white crystal by distilling off the solvent under reduced pressure, adding n-hexane and diisopropyl ether to the residue, filtering precipitated crystal and drying.

Melting point: 62 - 65 °C

Physical property values and the like about compounds of the general formula (1) prepared according to these processes are shown as follows. No.s in the Tables represent No.s of the compounds shown in the Tables 1 - 4. For example, the compound No.1-1 (a) is the compound No.1-1 of the formula (a) described in Table 1. Further, HCl described at the end of the compound No. means hydrochloride.

Table 5

| Compound No. | property (m.p.℃ etc.) |
|---|---|
| 1-1(a) | 95-96.5 |
| 1-2(a) | $n_D^{21.9}$ 1.4940 |
| 1-26(a) | $n_D^{21.9}$ 1.5284 |
| 1-39(a) | 97-98 |
| 1-52(a) | $n_D^{21.8}$ 1.5385 |
| 1-55(a) | $n_D^{21.9}$ 1.4934 |
| 1-66(a) | 43-44 |
| 1-93(a) | 165-168 |
| 1-113(a) | 178-180 |
| 1-126(a) | semi-crystal |
| 1-131(a) | semi-crystal |
| 1-171(a) | 101-103 |
| 1-176(a) | $n_D^{22.0}$ 1.5507 |
| 1-185(a) | 61-63 |
| 1-301(a) | $n_D^{21.9}$ 1.5381 |
| 2-1(a) | bp.95-101/4mmHg |
| 2-1(a)HCl | 215-216 |
| 2-1(f)HCl | 255-260 |
| 2-1(i)HCl | 170-175 |
| 2-2(a) | oil |
| 2-2(a)HCl | 173-176 |
| 2-3(a) | oil |
| 2-4(a) | oil |
| 2-5(a) | $n_D^{22.0}$ 1.4384 |
| 2-6(a) | oil |
| 2-7(a) | $n_D^{21.2}$ 1.4911 |
| 2-8(a) | semi-crystal |
| 2-10(a) | $n_D^{21.9}$ 1.4678 |
| 2-12(a) | $n_D^{21.3}$ 1.4726 |
| 2-15(a) | semi-crystal |
| 2-18(a) | $n_D^{21.8}$ 1.5112 |
| 2-22(a) | $n_D^{21.8}$ 1.5113 |
| 2-26(a) | 123-124 |
| 2-26(a)HCl | 145-148 |
| 2-52(a) | $n_D^{21.9}$ 1.5585 |
| 2-52(a)HCl | 215-220 |
| 2-60(a) | $n_D^{22.0}$ 1.5537 |
| 2-64(a) | oil |

Table 5 continued

| Compound No. | property (m.p.℃ etc.) |
|---|---|
| 2-64(a)HCl | 175-180 |
| 2-67(a) | $n_{D21.7}$ 1.4799 |
| 2-68(a) | 71-72 |
| 2-79(a) | oil |
| 2-87(a) | 90-92 |
| 2-91(a) | 128-130 |
| 2-104(a) | 193-195 |
| 2-126(a) | 133-134 |
| 2-130(a) | 190-195 |
| 2-136(a) | 55-56 |
| 2-172(a) | 59-61 |
| 2-183(a) | bp.114-115/1.5mmHg |
| 2-183(a)HCl | 202-204 |
| 2-184(a) | $n_{D22.0}$ 1.4982 |
| 2-185(a) | $n_{D21.5}$ 1.4201 |
| 2-186(a) | 96-98 |
| 2-187(a) | $n_{D21.9}$ 1.5449 |
| 2-195(a) | $n_{D21.8}$ 1.4938 |
| 2-199(a) | $n_{D21.8}$ 1.4832 |
| 2-204(a) | bp.101-101.5/5mmHg |
| 2-204(a)HCl | 175-178 |
| 2-205(a) | bp.113-114/0.3mmHg |
| 2-206(a) | bp.139.5~142.5/5mmHg |
| 2-206(a)HCl | 182-183 |
| 2-207(a) | $n_{D22.0}$ 1.5174 |
| 2-208(a) | $n_{D22.2}$ 1.4692 |
| 2-209(a) | $n_{D22.0}$ 1.4462 |
| 2-210(a) | $n_{D23.0}$ 1.5140 |
| 2-211(a) | $n_{D22.2}$ 1.4367 |
| 2-212(a) | $n_{D21.1}$ 1.5008 |
| 2-213(a) | $n_{D21.2}$ 1.5549 |
| 2-214(a) | 152-153 |
| 2-225(a) | 126-127 |
| 2-232(a) | 116-118 |
| 2-243(a)HCl | 126-128 |
| 2-256(a) | $n_{D21.8}$ 1.5049 |
| 2-267(a) | $n_{D21.8}$ 1.4880 |
| 2-278(a) | $n_{D23.2}$ 1.5132 |

Table 5 continued

| Compound No. | property (m.p.℃ etc.) |
|---|---|
| 2-308(a) | 184-185 |
| 2-342(a) | 157-158 |
| 2-361(a) | 124-125 |
| 2-383(a) | $n_{D}^{21.8}$ 1.5567 |
| 2-386(a) | $n_{D}^{21.8}$ 1.5525 |
| 2-400(a) | $n_{D}^{21.3}$ 1.5881 |
| 2-409(a) | $n_{D}^{21.8}$ 1.4609 |
| 2-413(a) | 51-52 |
| 2-414(a) | $n_{D}^{21.8}$ 1.5086 |
| 2-415(a) | bp.117-118/0.2mmHg |
| 2-415(a)HCl | 190-194 |
| 2-416(a) | $n_{D}^{21.8}$ 1.4771 |
| 2-417(a) | bp.118-119/0.2mmHg |
| 2-417(a)HCl | 181-187 |
| 2-418(a) | $n_{D}^{21.7}$ 1.5101 |
| 2-420(a) | $n_{D}^{21.3}$ 1.5167 |
| 2-424(a) | $n_{D}^{21.8}$ 1.4956 |
| 2-427(a) | $n_{D}^{21.9}$ 1.5153 |
| 2-428(a) | oil |
| 2-429(a) | 180-182 |
| 2-430(a) | oil |
| 2-431(a) | oil |
| 2-432(a) | oil |
| 2-433(i) | 97-98 |
| 2-436(a) | $n_{D}^{21.3}$ 1.5056 |
| 2-437(a) | $n_{D}^{21.3}$ 1.5014 |
| 2-450(a) | $n_{D}^{20.1}$ 1.5053 |
| 2-451(a) | $n_{D}^{21.5}$ 1.5114 |
| 2-453(a) | $n_{D}^{22.6}$ 1.5239 |
| 2-454(a) | $n_{D}^{21.7}$ 1.5210 |
| 2-456(a) | $n_{D}^{21.9}$ 1.5154 |
| 2-458(a) | 114-116 |
| 2-461(a) | $n_{D}^{21.0}$ 1.5140 |
| 2-462(a) | $n_{D}^{21.2}$ 1.5236 |
| 2-476(a) | $n_{D}^{21.6}$ 1.4972 |
| 2-480(a) | $n_{D}^{21.6}$ 1.4914 |
| 2-486(a) | $n_{D}^{21.6}$ 1.5166 |
| 3-1(a) | oil |

Table 5 continued

| Compound No. | property (m.p.℃ etc.) |
|---|---|
| 3-2(a) | 147-148 |
| 3-2(f) | 162-163 |
| 3-8(a) | 106.5-108 |
| 3-9(a) | $n_D^{21.9}$ 1.5460 |
| 3-10(a) | 111-112 |
| 3-14(a) | 109-110 |
| 3-17(a) | 104-105 |
| 3-19(a) | $n_D^{21.8}$ 1.5343 |
| 4-1(a) | 75-80 |
| 4-3(a) | 83-85 |
| 4-40(a) | oil |
| 4-41(a) | oil |
| 4-43(a) | $n_D^{24.5}$ 1.5767 |
| 4-48(a) | 84-86 |
| 4-54(a) | 153-156 |
| 4-60(a) | 80-100 |
| 4-79(a) | 169-171 |
| 4-80(a) | $n_D^{22.9}$ 1.5389 |
| 4-102(a) | 220 <decomposition> |
| 4-103(a) | 62-65 |
| 4-105(a) | 89-92 |
| 4-114(a) | 70-71 |
| 4-115(a) | 49-50 |
| 4-118(a) | 265-267 |
| 4-120(a) | 195-197 <decomposition> |
| 4-121(a) | 188-189 |
| 4-151(a) | $n_D^{20.6}$ 1.4173 |
| 4-154(a) | 132-134 |

Preparation examples of fungicide for agriculture and horticulture containing the compounds according to the invention as the active ingredient are shown as follows. However, the invention is not limited thereto. In the following preparation examples, "parts" means "parts by weight".

[Preparation example 1] Emulsion

| | |
|---|---|
| compound No.1-1 (a) according to the invention | 20 parts |
| methylnaphthalene | 55 parts |
| cyclohexanone | 20 parts |
| Solpol 2680 (mixture of nonionic surfactant and anionic surfactant: trade name of Toho Kagaku Kogyo Co. Ltd.) | 5 parts |

The above-mentioned components are mixed uniformly to make an emulsion. For use, the above-mentioned emulsion is diluted by 50 - 20000 times for applying 0.005 - 50 kg of the active ingredient per hectare.

[Preparation example 2] Wettable powder

| | |
|---|---|
| compound No.1-1 (a) according to the invention | 25 parts |
| pyrophyllite | 66 parts |
| Solpol 5039 (anionic surfactant: trade name of Toho Kagaku Kogyo Co. Ltd.) | 4 parts |
| Carplex #80D (white carbon: trade name of Shionogi Seiyaku Co. Ltd.) | 3 parts |
| calcium lignin sulfonate | 2 parts |

The above-mentioned components are mixed and grounded uniformly to make wettable powder. For use, the above-mentioned wettable powder is diluted by 50 - 20000 times for applying 0.005 - 50 kg of the active ingredient per hectare.

[Preparation example 3] Dust

| | |
|---|---|
| compound No.1-1(a) according to the invention | 3 parts |
| Carplex #80D (white carbon: trade name of Shionogi Seiyaku Co. Ltd.) | 0.5 parts |
| kaolinite | 95 parts |
| diisopropyl phosphate | 1.5 parts |

The above-mentioned components are mixed and grounded uniformly to make a dust. For use, the above-mentioned dusting agent is applied with 0.005 - 50 kg of the active ingredient per hectare.

[Preparation example 4] Granulate

| | |
|---|---|
| compound No.1-1 (a) according to the invention | 5 parts |
| bentonite | 30 parts |
| talc | 64 parts |
| calcium lignin sulfonate | 1 parts |

The above-mentioned components are mixed and grounded, and then stirred to mix with addition of a small amount of water, granulated by an extrusion granulator and dried to make granulates. For use, the above-mentioned granulate is applied with 0.005 - 50 kg of the active ingredient per hectare.

[Preparation example 5] Flowable powder

| | |
|---|---|
| compound No.1-1 (a) according to the invention | 25 parts |
| Solpol 3353 (nonionic surfactant: trade name of Toho Kagaku Kogyo Co. Ltd.) | 5 parts |
| Lunox 1000C (anionic surfactant: trade name of Toho Kagaku Kogyo Co. Ltd.) | 0.5 parts |
| xanthan gum (natural polymer) | 0.2 parts |
| sodium benzoate | 0.4 parts |
| propylene glycol | 10 parts |
| water | 58.9 parts |

The above-mentioned components except for the active ingredient (the compound according to the invention) are dissolved uniformly, to which the compound according to the invention is added, stirred well, and thereafter water-grounded in a sand mill to obtain flowable powders. For use, the above-mentioned flowable powder is diluted by 50 - 20000 times for applying 0.005 - 50 kg of the active ingredient per hectare.

[Pharmaceutical preparation example 6] Dry flowable powder

| | |
|---|---|
| compound No.1-1 (a) according to the invention | 75 parts |
| Hightenol NE-15 (anionic surfactant: trade name of Daiichi Kogyo Seiyaku Co. Ltd.) | 5 parts |
| Banilex N (anionic surfactant: trade name of Nihon Seishi Co. Ltd.) | 10 parts |
| Carplex #80D (white carbon: trade name of Shionogi Seiyaku Co. Ltd.) | 10 parts |

The above-mentioned components are mixed and grounded fine uniformly, and then stirred to mix with addition of a small amount of water, granulated by an extrusion granulator and dried to make dry flowable powder. For use, they are diluted by 50 - 20000 times for applying with 0.005 - 50 kg of the active ingredient per hectare.
Usefulness of the compounds according to the invention is illustrated specifically in the following test examples. However, it is not limited thereto.

[Test Example 1] Test for control of rice blast (water surface application)

Rice planted in a 1/20,000 are beaker pot was drenched at 1.5 leaf stage with 10 ml of agent solution which was prepared by diluting the emulsion of the compound according to the invention with water to 500 ppm.
14 days after application, treated rice was sprayed with a suspension of rice blast pathogen (Pyricularia oryzae) spores ($2x10^5$ /ml) for inoculation. Inoculated rice was then placed in an inoculation box at a temperature of 20 - 25 °C and a humidity of 95 % or more for one day and night. Thereafter, the rice was placed in a greenhouse and evaluated 7 days after inoculation for an extent of disease by 6 indices, 0-5.

(Evaluation standard)

| disease index | Extent of disease |
|---|---|
| 0 | no disease |
| 1 | disease spots 1 - 5 |
| 2 | disease spots 6 - 20 |
| 3 | disease spots 21 - 40 |
| 4 | disease spots 41 - 70 |
| 5 | disease spots 71 or more |

As a result following compounds exhibited disease index 1; 1-2(a), 1-26(a), 1-52(a), 1-55(a), 1-93(a), 1-113(a), 1-126(a), 1-131(a), 1-171(a), 1-176(a), 1-185(a), 2-1(a)HCl, 2-1(i)HCl, 2-2(a), 2-2(a)HCl, 2-3(a), 2-4(a), 2-5(a), 2-6(a), 2-7(a), 2-8(a), 2-10(a), 2-15(a), 2-18(a), 2-22(a), 2-26(a), 2-26(a)HCl, 2-36(a), 2-52(a), 2-52(a)HCl, 2-60(a), 2-64(a), 2-64(a)HCl, 2-67(a), 2-68(a), 2-79(a), 2-87(a), 2-91(a), 2-126(a), 2-130(a), 2-136(a), 2-172(a), 2-183(a), 2-183(a)HCl, 2-184(a), 2-185(a), 2-186(a), 2-187(a), 2-195(a), 2-199(a), 2-204(a), 2-204(a)HCl, 2-205(a), 2-206(a), 2-206(a)HCl, 2-207(a), 2-208(a), 2-209(a), 2-210(a), 2-211(a), 2-212(a), 2-213(a), 2-214(a), 2-225(a), 2-232(a), 2-243(a)HCl, 2-256(a), 2-342(a), 2-361(a), 2-383(a), 2-386(a), 2-400(a), 2-409(a), 2-413(a), 2-414(a), 2-415(a), 2-415(a)HCl, 2-416(a), 2-417(a), 2-417(a)HCl, 2-418(a), 2-420(a), 2-424(a), 2-426(a), 2-432(a), 2-436(a), 2-437(a), 2-450(a), 2-453(a), 2-454(a), 2-456(a), 2-458(a), 2-471(a), 2-480(a), 2-486(a), 3-1(a), 3-2(a), 3-2(f), 3-8(a), 3-9(a), 3-10(a), 3-14(a), 3-17(a), 3-19(a), 4-105(a), 4-114(a), 4-115(a), 4-118(a), 4-151(a), 4-120(a).

[Test Example 2] Test for control of cucumber downy mildew

Cucumber (species: Sagami Hanjiro) grown in a pot having a diameter of 7 cm was applied at 1.5 leaf stage with 20 ml per a pot of agent solution which was prepared by diluting the emulsion of the compound according to the invention with water to 500 ppm by means of a spray-gun.
5 days after application, suspension of cucumber downy midlew pathogen (Pseudoperonospora cubensis) spores ($2x10^5$ /ml) for inoculation, Inoculated cucumber was placed in an inoculation box at a temperature of 20 - 25 °C and a humidity of 95 % or more for one day and night. Thereafter, the cucumber was placed in a greenhouse and determined a ratio of disease spot area formed after 7 days from inoculation to inoculated leaves to calculate a control value according to the following equation:

control value = [1-(disease spot area proportion in treated part/ disease spot area proportion in non-treated part)] x 100

As the result, the following compounds exhibited control value of 70 or higher:

compounds No.; 1-1(a), 2-1(f), 3-2(f).

[Test Example 3] Test for control of barley powdery mildew

Barley (species: Saitama Sekitori No.2) grown in a pot having a diameter of 5.5 cm was applied at 2.5 leaf stage with 20 ml per a pot of agent solution which was prepared by diluting the emulsion of the compound according to the invention with water to 500 ppm by means of a spray-gun.

7 days after spraying, barley powderly mildew pathogen (Erysiphe graminis) spores were inoculated directly. Thereafter, the barley was placed in a greenhouse and determined a ratio of disease spot area formed after 14 days from inoculation to inoculated leaves to calculate a control value according to the following equation:

control value = [1-(disease spot area proportion in treated part/ disease spot area proportion in non-treated part)] x 100

As the result, the following compounds exhibited control value of 70 or higher:

compounds No.; 2-26(a).

[Test Example 4] Test for control of wheat powdery mildew

Wheat (species: Norin No.61) grown in a pot having a diameter of 5.5 cm was sprayed at 2.0 - 2.5 leaf stage by means of a spray-gun with 20 ml per a pot of agent solution which was prepared by diluting the emulsion of the compound according to the invention with water to 500 ppm.

7 days after spraying, wheat powdery mildew pathogen (Erysiphe graminis) spores were inoculated directly. Thereafter, the wheat was placed in a greenhouse and determined a ratio of disease spot area formed after 10 days from inoculation to inoculated leaves to calculate a control value according to the following equation:

control value = [1-(disease spot area proportion in treated part/ disease spot area proportion in non-treated part)] x 100

As the result, the following compounds exhibited control value of 70 or higher:

compounds No.; 1-131(a), 1-171(a), 2-1(a), 2-1(f), 2-15(a), 2-68(a), 2-126(a), 2-187(a), 2-256(a), 2-386(a).

[Test Example 5] Test for control of wheat brown rust

Wheat (species: Norin No.61) grown in a pot having a diameter of 5.5 cm was sprayed at 2.0 - 2.5 leaf stage with 20 ml per a pot of agent solution which was prepared by diluting the emulsion of the compound according to the invention with water to 500 ppm by means of a spray-gun.

7 days after spraying, a suspension of wheat brown rust pathogen (Puccinia recondita) spores ($2x10^5$ /ml) for inoculation, placed in an inoculation box at a temperature of 20-25 °C and a humidity of 95 % or more for one day and night. Thereafter, the wheat was placed in a greenhouse and determined a ratio of disease spot area formed after 10 days from inoculation to inoculated leaves to calculate a control value according to the following equation:

control value = [1-(disease spot area proportion in treated part/ disease spot area proportion in non-treated part)] x 100

As the result, the following compounds exhibited control value of 70 or higher:

compounds No.; 2-1(a) HCl.

<u>Effect of the Invention</u>

These compounds according to the invention have superior plant disease control actions and they are safe for crops.

**Claims**

1. A pyrazole compound of the general formula (1):

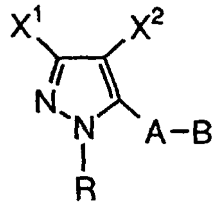

(1)

wherein,

R is $C_1$-$C_4$ alkyl,
$X^1$ is halogen,
$X^2$ is H or halogen,
A is $[CH(R^1)]_g$, $C(R^1)= C(R^2)$ or $C[=C(R^1)(R^2)]$,
g is 0, 1 or 2,
$R^1$ is H, $C_1$-$C_4$ alkyl or phenyl optionally substituted with Ra,
$R^2$ is H, CN, $C_1$-$C_4$ alkyl, $C_1$-$C_4$ alkoxy, $C_1$-$C_4$ alkylthio, $C_1$-$C_4$ alkylsulfinyl, $C_1$-$C_4$ alkylsulfonyl, $C_1$-$C_4$ alkoxy-carbonyl or aryl optionally substituted with Ra,
B is, when g is 1 or 2 in the definition of A, $N_3$, OH, $NH_2$, CN, CHO, $OR^3$, $S(O)_d$ $R^3$, $NHS(O)_2R^3$, $N(R^4)OR^3$, $S(O)_2N(R^5)(R^6)$, $N(R^7)(R^8)$, $ON(R^7)(R^8)$, $ON=C(R^9)(R^{10})$, $N(R^4)N(R^5)(R^6)$, $CO_2R^4$, $C(R^{23})=NR^{24}$, $C(R^{23})=NOR^{24}$, $C(R^{23})=NN(R^5)(R^6)$, $C(R^{23})=NN=C(R^9)(R^{10})$.

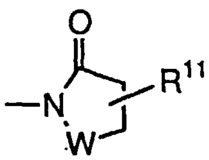

B1 ,

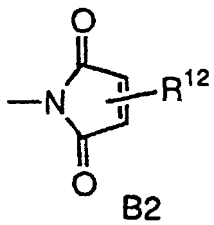

B2 ,

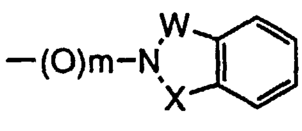

B3 ,

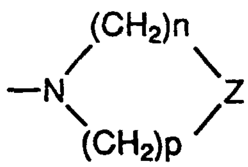

B4 ,

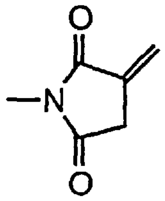

B5

,

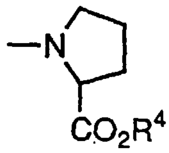

B6

,

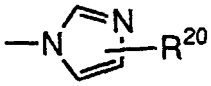

B7

,

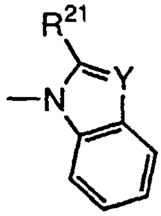

B8

,

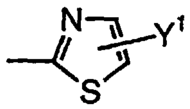

B9

,

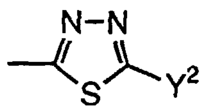

B10

,

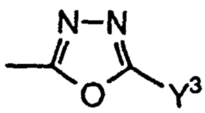

B11

,

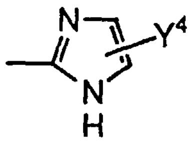

B12

,

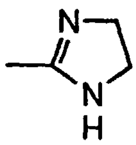

B13

,

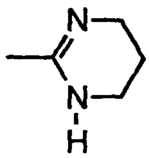

B14

,

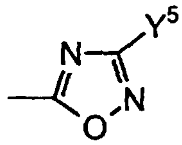

B15

,

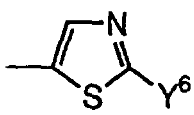

B16

,

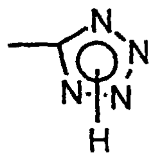

or

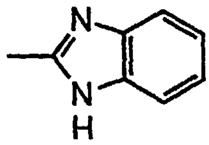

B17 B18

or

B is, when g is 0 in the definition of A, $C(R^{23})=NR^{24}$, $C(R^{23})=NOR^{24}$, $C(R^{23})=NN=(R^5)(R^6)$, $C(R^{23})=NN=C(R^9)(R^{10})$, B9, B10, B11, B12, B13, B14, B15, B16, B17 or B18.

d is 0, 1 or 2,

m is 0 or 1,

n is 1 or 2,

p is 1, 2 or 3,

$R^3$ is $C_1$-$C_{12}$ alkyl, $C_1$-$C_{12}$ haloalkyl, $C_2$-$C_{10}$ alkenyl, $C_2$-$C_{10}$ haloalkenyl, $C_2$-$C_{10}$ alkynyl, $C_3$-$C_{10}$ cycloalkyl or $[C(R^4)(R^{14})]_f$-$R^{15}$,

f is 0, 1 or 2,

$R^4$ is H or $C_1$-$C_4$ alkyl,

$R^5$ and $R^6$ are each independently H, $C_1$-$C_{12}$ alkyl, $C_2$-$C_{10}$ alkenyl, aryl optionally substituted with Ra or benzyl optionally substituted with Ra, or $R^5$ and $R^6$ together are $C_3$-$C_6$ alkylene chain,

$R^7$ is H, $C_1$-$C_{12}$ alkyl, $C_1$-$C_{12}$ haloalkyl, $C_2$-$C_{10}$ alkenyl, $C_3$-$C_{10}$ cycloalkyl or $[C(R^4)(R^{14})]_f$-$R^{15}$,

$R^8$ is $C_1$-$C_{12}$ alkyl, $C_1$-$C_{12}$ haloalkyl, $C_2$-$C_{10}$ alkenyl, $C_3$-$C_{10}$ cycloalkyl, $C_1$-$C_4$ alkylamino-$C_2$-$C_4$ alkyl, di($C_1$-$C_4$ alkyl)amino-$C_2$-$C_4$ alkyl, $C_1$-$C_4$ alkoxy-$C_2$-$C_4$ alkyl or $[C(R^4(R^{14})]_f$-$R^{15}$, or $R^7$ and $R^8$ together are $C_3$-$C_7$ alkylene chain optionally substituted with OH or $C_1$-$C_4$ alkyl,

$R^9$ and $R^{10}$ are each independently H, $C_1$-$C_{12}$ alkyl, $C_2$-$C_{10}$ alkenyl, $C_3$-$C_{10}$ cycloalkyl, aryl optionally substituted with Ra or benzyl optionally substituted with Ra, or $R^9$ and $R^{10}$ together are $C_3$-$C_6$ alkylene chain,

$R^{11}$ is H or $C_1$-$C_6$ alkyl,

W is direct bond, C(O), $SO_2$, $CH_2$, $CH_2CH_2$ or $CH_2CH_2CH_2$,

$R^{12}$ is H, halogen or $C_1$-$C_6$ alkyl,

X is C(O), $SO_2$, $CH_2$, $CH_2CH_2$, or $CH_2CH_2CH_2$,

Z is O, S, $CH(CH_3)OCH(CH_3)$, CH=CH, $N(R^{13})$ or $CH(CH_3)N(R^{13})CH(CH_3)$,

Y is N or $C(R^{22})$,

$R^{13}$ is H, CHO, $C_1$-$C_6$ alkyl, $C_2$-$C_{10}$ alkenyl, $C_1$-$C_6$ alkoxycarbonyl, $C_3$-$C_{10}$ cycloalkyl, $C_1$-$C_6$ alkylcarbonyl, aryl or benzyl,

$R^{14}$ is H, $C_1$-$C_6$ alkyl, $C_1$-$C_6$ alkylthio-$C_2$-$C_4$ alkyl, aryl optionally substituted with Ra or benzyl optionally substituted with Ra,

$R^{15}$ is aryl optionally substituted with Rb, CN, $C(Q)R^{16}$, $C(Q)OR^{17}$ or $C(Q)N(R^{18})(R^{19})$.

Q is O or S,

$R^{16}$ is H, $C_{1-12}$ alkyl, $C_1$-$C_{12}$ haloalkyl, $C_2$-$C_{10}$ alkenyl, $C_3$-$C_{10}$ cycloalkyl, aryl optionally substituted with Rb or benzyl optionally substituted with Rb,

$R^{17}$ is H, $C_1$-$C_6$ alkyl, $C_2$-$C_{10}$ alkenyl, aryl optionally substituted with Ra or benzyl optionally substituted with Ra,

$R^{18}$ and $R^{19}$ each independently H, $C_1$-$C_6$ alkyl, aryl optionally substituted with Ra or benzyl optionally substituted with Ra, or $R^{18}$ and $R^{19}$ together are $C_3$-$C_6$ alkylene chain,

$R^{20}$ is H, halogen, $C_1$-$C_4$ alkyl or phenyl,

$R^{21}$ is H, halogen, $C_1$-$C_4$ alkyl or $C_1$-$C_4$ alkylthio,

$R^{22}$ is H, halogen or $C_1$-$C_4$ alkyl,

$R^{23}$ is H, $C_1$-$C_4$ alkyl or phenyl optionally substituted with Ra,

$R^{24}$ is H, $C_1$-$C_6$ alkyl, $C_2$-$C_{10}$ alkenyl, $C_2$-$C_{10}$ haloalkenyl, $CH_2CO_2R^4$, phenyl optionally substituted with Ra or benzyl optionally substituted with Ra,

$Y^1$ is H, $C_1$-$C_4$ alkyl, $C_1$-$C_4$ alkoxycarbonyl or phenyl optionally substituted with Ra,

$Y^2$ is H, $C_1$-$C_4$ alkyl or phenyl optionally substituted with Ra,

$Y^3$ is H, $C_1$-$C_4$ alkyl, SH, $C_1$-$C_4$ alkylthio or phenyl optionally substituted with Ra,

$Y^4$ is H, $C_1$-$C_4$ alkyl or phenyl optionally substituted with Ra,

$Y^5$ is H, $C_1$-$C_4$ alkyl, $C_1$-$C_4$ haloalkyl or phenyl optionally substituted with Ra,
$Y^6$ as H, $NH_2$, $C_1$-$C_4$ alkyl or phenyl optionally substituted with Ra,
Ra, being same or different, is 1 - 5 substituent(s) selected from halogen, $NO_2$, CN, $C_1$-$C_4$ alkyl or $C_1$-$C_4$ alkoxy,
Rb, being same or different, is 1 - 5 substituent(s) selected from halogen, $NO_2$, CN, $NH_2$, OH, $C_1$-$C_4$ alkyl, $C_1$-$C_4$ alkoxy, $C_1$-$C_4$ haloalkyl, carboxyl or $C_1$-$C_4$ alkoxycarbonyl,
aryl is phenyl or naphthyl.

**2.** A pyrazole compound according to claim 1, wherein $X^1$ is Cl or Br, $X^2$ is H or Cl, R is methyl, ethyl, n- or i-propyl or n-butyl.

**3.** A pyrazole compound according to claim 1, wherein $X^1$ is Cl or Br, $X^2$ is H or Cl, R is methyl, ethyl, n- or i-propyl or n-butyl, A is $(CH(R^1)]_g$ or $C(R^1)=C(R^2)$.

**4.** A pyrazole compound according to claim 1, wherein $X^1$ is Cl or Br, $X^2$ is H or Cl, R is methyl, A is $[CH(R^1)]_g$ or $C(R^1)=C(R^2)$.

**5.** A pyrazole compound according to claim 1, wherein $X^1$ is Cl, $X^2$ is H or Cl, R is methyl, A is $[CH(R^1)]_g$.

**6.** A pyrazole compound according to claim 1, wherein $X^1$ is Cl, $X^2$ is H or Cl, R is methyl, A is $[CH(R^1)]_g$, g is 1, $R^1$ is H or methyl.

**7.** A pyrazole compound according to claim 1, wherein $X^1$ is Cl, $X^2$ is H or Cl, R is methyl, A is $CH_2$.

**8.** A pyrazole compound according to claim 1, wherein $X^1$ is Cl, $X^2$ is H or Cl, R is methyl, A is $CH_2$, B is $NH_2$, OH, $OR^3$, $S(O)_dR^3$, $NHS(O)_2R^3$, $N(R^4)OR^3$, $S(O)_2N(R^5)(R^6)$, $N(R^7)(R^8)$, $ON(R^7)(R^8)$, $ON=C(R^9)(R^{10})$, $N(R^4)N(R^5)(R^6)$, B3, B4 or B7.

**9.** A pyrazole compound according to claim 1, wherein $X^1$ is Cl, $X^2$ is H, R is methyl, A is $CH_2$, B is $NH_2$, OH, $OR^3$, $N(R^7)(R^8)$, $ON=C(R^9)(R^{10})$, $N(R^4)N(R^5)(R^6)$, B3, B4 or B7.

**10.** A pyrazole compound according to claim 1, wherein $X^1$ is Cl, $X^2$ is H, R is methyl, A is $CH_2$, B is $NH_2$, $N(R^7)(R^8)$, $N(R^4)N(R^5)(R^6)$, B3, B4 or B7.

**11.** A pyrazole compound according to claim 1, wherein $X^1$ is Cl, $X^2$ is H, R is methyl, A is $CH_2$, B is $N(R^7)(R^8)$ or B4.

**12.** A pyrazole compound according to claim 1, wherein $X^1$ is Cl, $X^2$ is H, R is methyl, A is $CH_2$, B is $NH_2$ or $N(R^7)(R^8)$, $R^7$ is H, methyl, ethyl, n- or i-propyl, n-, i-, s-or t-butyl, allyl, phenyl or benzyl, $R^8$ is methyl, ethyl, n- or i-propyl, n-, i-, s- or t-butyl, allyl, phenyl or benzyl, or $R^7$ and $R^8$ together are $(CH_2)_4$, $(CH_2)_5$ or $(CH_2)_6$.

**13.** A pyrazole compound according to claim 1, wherein $X^1$ is Cl, $X^2$ is H, R is methyl, A is $CH_2$, B is B4, n is 1 or 2, p is 1 or 2, Z is CH=CH, $N(R^{13})$ or $CH(CH_3)N(R^{13})CH(CH_3)$, $R^{13}$ is H, methyl, ethyl, n- or i-propyl, allyl, benzyl or phenyl.

**14.** A pyrazole compound according to claim 1, wherein $X^1$ is Cl, $X^2$ is H, R is methyl, A is $CH_2$, B is $NH_2$, methylamino, ethylamino, dimethylamino, diethylamino, N-ethyl-n-propylamino, n-propylamino, i-propylamino, di(n-propyl)amino, n-butylamino, i-butylamino, s-butylamino, t-butylamino, di(i-butyl)amino, allylamino, diallylamino, anilino, benzylamino, pyrrolidino, piperidino, hexamethyleneimino, piperazino, N-methylpiperazino, N-ethylpiperazino, N-(n-propyl)piperazino, N-allylpiperazino, N-phenylpiperazino, N-benzylpiperazino or N-(1,2,3,6-tetrahydropyridino).

**15.** A pyrazole compound selected from the group consisting of

(1) 3-chloro-1-methyl-5-(dimethylamino)methyl pyrazole,
(2) 3-chloro-1-methyl-5-(diethylamino)methyl pyrazole,
(3) 3-chloro-1-methyl-5-(N-ethyl-n-propylamino)methyl pyrazole,
(4) 3-chloro-1-methyl-5-(n-propylamino)methyl pyrazole,
(5) 3-chloro-1 -methyl-5-(i-butylamino)methyl pyrazole,
(6) 3-chloro-1-methyl-5-(s-butylamino)methyl pyrazole,

(7) 3-chloro-1 -methyl-5-(di-i-butylamino)methyl pyrazole,
(8) 3-chloro-1-methyl-5-(allylamino)methyl pyrazole,
(9) 3-chloro-1-methyl-5-(diallylamino)methyl pyrazole,
(10) 3-chloro-1-methyl-5-(benzylamino)methyl pyrazole,
(11) 3-chloro-1-methyl-5-(pyrrolidino)methyl pyrazole,
(12) 3-chloro-1-methyl-5-(hexamethyleneimino)methyl pyrazole,
(13) 3-chloro- 1-methyl-5-(1-piperadino)methyl pyrazole,
(14) 3-chloro-1-methyl-5-(4-methyl-1-piperadino)methyl pyrazole,
(15) 3-chloro-1-methyl-5-(4-ethyl-1-piperadino)methyl pyrazole,
(16) 3-chloro-1-methyl-5-(4-allyl-1-piperadino)methyl pyrazole,
(17) 3-chloro-1-methyl-5-(4-phenyl-1-piperadino)methyl pyrazole,
(18) 3-chloro-1-methyl-5-(4-benzyl-1-piperadino)methyl pyrazole,
(19) (3-chloro-1-methylpyrazol-5-yl)methylamine,
(20) 3-chloro-1-methyl-5-(methylamino)methyl pyrazole and
(21) 3-chloro-1-methyl-5-(ethylamino)methyl pyrazole.

**16.** A hydrochloride, hydrobromide, hydroiodide, formate, acetate or oxalate of a pyrazole compound according to any one of claims 1 to 15.

**17.** A Plant disease control agent containing at least one compound or its salt according to any one of claims 1 to 16 as active ingredient.

# INTERNATIONAL SEARCH REPORT

International application No.
PCT/JP98/03997

A. CLASSIFICATION OF SUBJECT MATTER
Int.Cl[6] C07D231/16, 401/06, 403/06, A01N43/56, 43/34, 43/72

According to International Patent Classification (IPC) or to both national classification and IPC

B. FIELDS SEARCHED

Minimum documentation searched (classification system followed by classification symbols)
Int.Cl[6] C07D231/16, 401/06, 403/06, A01N43/56, 43/34, 43/72

Documentation searched other than minimum documentation to the extent that such documents are included in the fields searched

Electronic data base consulted during the international search (name of data base and, where practicable, search terms used)
REGISTRY (STN), CA (STN)

C. DOCUMENTS CONSIDERED TO BE RELEVANT

| Category* | Citation of document, with indication, where appropriate, of the relevant passages | Relevant to claim No. |
|---|---|---|
| A | JP, 2-4779, A (Bayer AG.), 9 January, 1990 (09. 01. 90) & EP, 335156, A & DE, 3810382, A | 1-17 |
| A | JP, 2-53775, A (Nissan Chemical Industries, Ltd.), 22 February, 1990 (22. 02. 90) (Family: none) | 1-17 |
| A | JP, 163063, A (Nihon Nohyaku Co., Ltd.), 15 July, 1991 (15. 07. 91) (Family: none) | 1-17 |

☐ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 24 November, 1998 (24. 11. 98) | 8 December, 1998 (08. 12. 98) |

| Name and mailing address of the ISA/ | Authorized officer |
|---|---|
| Japanese Patent Office | |
| Facsimile No. | Telephone No. |

Form PCT/ISA/210 (second sheet) (July 1992)